# EUROPÄISCHE PATENTSCHRIFT

(11) **EP 1 664 039 B1**
(45) Veröffentlichungstag und Bekanntmachung des Hinweises auf die Patenterteilung: **05.03.2014**
(21) Anmeldenummer: 04764704.5
(22) Anmeldetag: 01.09.2004
(51) Int. Cl.: C07D 413/12, C07D 263/58, A61K 31/423, A61P 35/00

(54) **1,3-BENZOXAZOLYLDERIVATE ALS KINASE-INHIBITOREN**
1,3-BENZOXAZOLYL DERIVATIVES AS KINASE-INHIBITORS
DERIVES DE 1,3-BENZOXAZOLYLE SERVANT D'INHIBITEURS DE KINASE

(30) Priorität: 24.09.2003 DE 10344223
(43) Veröffentlichungstag der Anmeldung: 07.06.2006
(73) Patentinhaber: Merck Patent GmbH, 64293 Darmstadt (DE)
(72) Erfinder: STÄHLE, Wolgang, 55218 Ingelheim (DE); JONCZYK, Alfred, 64295 Darmstadt (DE); RAUTENBERG, Wilfried, 64354 Reinheim (DE)
(86) Internationale Anmeldenummer: PCT/EP2004/009743
(87) Internationale Veröffentlichungsnummer: WO 2005/037829

(56) Entgegenhaltungen:
- WO-A-02/44156
- WO-A-02/076986
- WO-A-02/080926

## Beschreibung

### Hintergrund der Erfindung

Die Erfindung betrifft Verbindungen der Formel I, 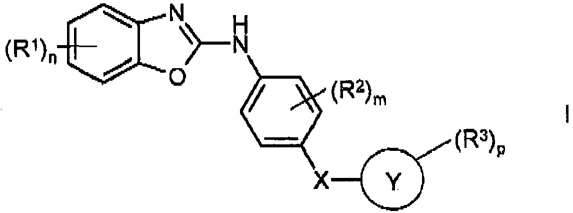 worin
- R¹, R², R³: jeweils unabhängig voneinander R, Hal, CN, NO₂, NHR, NRR, NHCOR, NHSO₂R, OR, CO-R, CO-NHR, CF₃, OCF₃, SCF₃, SO₃R, SO₂R, SO₂NR, SR, COOH oder COOR,
- R: H,
- A: unverzweigtes, verzweigtes oder cyclisches Alkyl mit 1-14 C-Atomen, worin eine oder zwei CH₂-Gruppen durch O- oder S-Atome und/oder durch -CH=CH-Gruppen und/oder auch 1-7 H-Atome durch F und/oder Cl ersetzt sein können,
- Ar: unsubstituiertes oder ein-, zwei- oder dreifach durch A, Hal, OH, OA, CN, NO₂, NH₂, NHA, NA₂, NHCOA, SCF₃, SO₂A, COOH, COOA, CONH₂, CONHA, CONA₂, NHSO₂A, SO₂NH₂, SO₂NHA, SO₂NA₂, CHO oder COA substituiertes Phenyl, Naphthyl oder Biphenyl,
- Het: einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Carbonylsauerstoff, Hal, A, -(CH₂)_{b}-Ar, -(CH₂)_{b}-Cycloalkyl, OH, OA, NH₂, NHA, NA₂, NO₂, CN, COOH, COOA, CONH₂, CONHA, CONA₂, NHCOA, NHCONH₂, NHSO₂A, CHO, COA, SO₂NH₂ und/oder S(O)_{g}A substituiert sein kann,
- Hal: F, Cl, Br oder I,
- X: O, S, SO₂NH oder NH,
- Ⓨ: Phenyl oder einen monocyclischen aromatischen Heterocyclus mit 1 bis 4 N-, O- und/oder S-Atomen,
- b,: 0, 1, 2, 3 oder 4,
- g: 0, 1 oder 2,
- n, m, p, q: jeweils unabhängig voneinander 1, 2, 3, oder 4
bedeuten,
sowie ihre pharmazeutisch unbedenklichen Salze, Solvate und Stereoisomere, einschließlich deren Mischungen in allen Verhältnissen.

Es wurde gefunden, dass die Verbindungen der Formel I die Signaltransduktion, die durch Kinasen vermittelt wird, insbesondere durch Tyrosinkinasen und/oder Raf-Kinasen, hemmen; regulieren und/oder modulieren können. Insbesondere eigenen sich die erfindungsgemäßen Verbindungen als Inhibitoren von Tyrosinkinasen und/oder Raf-Kinasen. So können erfindungsgemäße Medikamente und pharmazeutische Zusammensetzungen wirksam zur Behandlung von Krankheiten eingesetzt werden, die durch Kinasen und/oder durch kinase-vermittelte Signaltransduktion bzw. durch Angiogenese verursacht, vermittelt und/oder propagiert werden. Somit eigenen sich die erfindungsgemäßen Verbindungen zur Behandlung und Prophylaxe von Krebs, Tumorwachstum, Arteriosklerose, altersbedingter Makula-Degeneration, diabetischer Retinopathie, Entzündungserkrankungen und dergleichen bei Säugetieren.

Bei den Tyrosinkinasen handelt es sich um eine Klasse von Enzymen, die die Übertragung des endständigen Phosphats des Adenosintriphosphats auf Tyrosinreste bei Proteinsubstraten katalysieren. Man nimmt an, dass den Tyrosinkinasen bei verschiedenen Zellfunktionen über die Substrat-phosphorylierung eine wesentliche Rolle bei der Signaltransduktion zukommt. Obwohl die genaue Mechanismen der Signaltransduktion noch unklar sind, wurde gezeigt, dass die Tyrosinkinasen wichtige Faktoren bei der Zellproliferation, der Karzinogenese und der Zelidifferenzierung darstellen.
Die Tyrosinkinasen lassen sich in Rezeptor-Tyrosinkinasen und zytosolische Tyrosinkinasen einteilen. Die Rezeptor-Tyrosinkinasen weisen einen extrazellulären Teil, einen Transmembranteil und einen intrazellulären Teil auf, während die zytosolischen Tyrosinkinasen ausschließlich intrazellulär vorliegen.

Die Rezeptor-Tyrosinkinasen bestehen aus einer Vielzahl von Transmembranrezeptoren mit unterschiedlicher biologischer Wirksamkeit. So wurden ungefähr 20 verschiedene Unterfamilien von Rezeptor-Tyrosin-kinasen identifiziert. Eine Tyrosinkinase-Unterfamilie, die die Bezeichnung EGFR- oder HER-Unterfamilie trägt, besteht aus EGFR, HER2, HER3 und HER4. Zu den Liganden dieser Rezeptor-Unterfamilie zählen der Epithel-Wachstumsfaktor (EGF), der Gewebewachstumsfaktor (TGF-α), Amphiregulin, HB-EGF, Betacellulin und Heregulin. Die Insulin-Unterfamilie, zu der INS-R, IGF-IR und IR-R zählen, stellt eine weitere Unterfamilie dieser Rezeptor-Tyrosinkinasen dar. Die PDGF-Unterfamilie beinhaltet den PDGF-α- and -β-Rezeptor, CSFIR, c-kit und FLK-II. Außerdem gibt es die FLK-Familie, die aus dem Kinaseinsertdomänenrezeptor (KDR) oder VEGFR-2, der fötalen Leberkinase-1 (FLK-1), der fötalen Leberkinase-4 (FLK-4) und der fms-Tyrosinkinase-1 (flt-1) oder VEGFR-1 besteht. Die PDGF- und FLK-Familie werden üblicherweise aufgrund der zwischen den beiden Gruppen bestehenden Ähnlichkeiten in der Gruppe der Splitkinase-Domänen Rezeptor-Tyrosinkinasen zusammengefasst (Laird, A. D. und J. M. Cherrington, Expert. Opin. Investig. Drugs 12(1): 51-64, 2003). Für eine genaue Diskussion der Rezeptor-Tyrosinkinasen siehe die Arbeit von Plowman et al., DN & P 7(6):334-339, 1994, die hiermit durch Bezugnahme aufgenommen wird.

Die zytosolischen Tyrosinkinasen bestehen ebenfalls aus einer Vielzahl von Unterfamilien, darunter Src, Frk, Btk, Csk, Abl, Zap70, Fes/Fps, Fak, Jak, Ack, and LIMK. Jede dieser Unterfamilien ist weiter in verschiedene Untergruppen unterteilt. So stellt zum Beispiel die Src-Unterfamilie eine der größten Unterfamilien dar. Sie beinhaltet Src, Yes, Fyn, Lyn, Lck, Blk, Hck, Fgr und Yrk. Die Src-Enzymunterfamilie wurde mit der Onkogenese in Verbindung gebracht. Für eine genauere Diskussion der zytosolischen Tyrosinkinasen, siehe die Arbeit von Bolen, Oncogene, 8:2025-2031 (1993), die hiermit durch Bezugnahme aufgenommen wird. Sowohl die Rezeptor-Tyrosinkinasen als auch die zytosolischen Tyrosin-kinasen sind an Signalübertragungswegen der Zelle, die zu Leidenszuständen wie Krebs, Schuppenflechte und Hyperimmunreaktionen führen, beteiligt.

Krebs ist eine Krankheit, deren Ursachen in einer gestörten Signaltransduktion zu sehen sind. Insbesondere deregulierte Signaltransduktion über Tyrosinkinasen spielt eine Hauptrolle beim Wachstum und der Ausbreitung von Krebs (Blume-Jensen, P. und T. Hunter, Nature 411: 355-365, 2001; Hanahan D. und R. A. Weinberg, Cell 100:57-70, 2000). Tyrosinkinasen und insbesondere Rezeptor-Tyrosinkinasen sowie die an sie bindenden Wachstumsfaktoren können so an deregulierter Apoptose, Gewebeinvasion, Metastasierung und allgemein an Signaltransduktionsmechanismen, die zu Krebs führen, beteiligt sein.

Rezeptor-Tyrosinkinasen spielen insbesondere eine Rolle bei der Angiogenese, ein weiterer wichtiger Mechanismus beim Wachstum und Ausbreitung von Krebs (Mustonen und Alitalo, J. Cell Biol. 129:895-898, 1995). Eine dieser Rezeptor-Tyrosinkinasen ist die fötale Leberkinase 1, auch FLK-1 genannt. Das menschliche Analog der FLK-1 ist der kinaseinsert-domänenhaltige Rezeptor KDR, der auch unter der Bezeichnung Gefäßendothelzellenwachstumsfaktorrezeptor 2 bzw. VEGFR-2 bekannt ist, da er VEGF hochaffin bindet. Die Maus-Version dieses Rezeptors wurde NYK genannt (Oelrichs et al., Oncogene 8(1):11-15, 1993). VEGF und KDR stellen ein Liganden-Rezeptor-Paar dar, das eine wesentliche Rolle bei der Proliferation der Gefäßendothelzellen und der Bildung und Sprossung der Blutgefäße, die als Vaskulogenese bzw. Angiogenese bezeichnet werden, spielt.
Die Angiogenese ist durch eine übermäßig starke Aktivität des Gefäßendothelwachstumsfaktors (VEGF) gekennzeichnet. Der VEGF besteht eigentlich aus einer Familie von Liganden (Klagsburn und D'Amore, Cytokine & Growth Factor Reviews 7:259-270, 1996). Der VEGF bindet den hochaffinen transmembranösen Tyrosinkinaserezeptor KDR und die verwandte fms-Tyrosinkinase-1, auch unter der Bezeichnung Flt-1 oder Gefäßendothelzellenwachstumsfaktorrezeptor 1 (VEGFR-1) bekannt. Aus Zellkultur- und Gen-Knockout-Versuchen geht hervor, dass jeder Rezeptor zu unterschiedlichen Aspekten der Angiogenese beiträgt. Der KDR führt die mitogene Funktion des VEGF herbei, während Flt-1 nicht-mitogene Funktionen, wie diejenigen, die mit der Zelladhäsion in Zusammenhang stehen, zu modulieren scheint. Eine Hemmung des KDR moduliert daher das Niveau der mitogenen VEGF-Aktivität. Tatsächlich wurde gezeigt, dass das Tumorwachstum von der antiangiogenen Wirkung der VEGF-Rezeptor-Antagonisten beeinflusst wird (Kim et al., Nature 362, S. 841-844,1993).

Die VEGF-Expression ist auch in hypoxischen Regionen von tierischen und menschlichen Tumoren neben Nekrosezonen stark erhöht. Der VEGF wird außerdem durch die Expression der Onkogene ras, raf, src und p53-Mutante (die alle bei der Bekämpfung von Krebs von Bedeutung sind) hinaufreguliert. Monoklonale Anti-VEGF-Antikörper hemmen bei der Nacktmaus das Wachstum menschlicher Tumore. Obwohl die gleichen Tumorzellen in Kultur weiterhin VEGF exprimieren, verringern die Antikörper ihre Zellteilungsrate nicht. So wirkt der aus Tumoren stammende VEGF nicht als autokriner mitogener Faktor. Der VEGF trägt daher in vivo dadurch zum Tumorwachstum bei, dass er durch seine parakrine Gefäßendothelzellen-Chemotaxis- und -Mitogeneseaktivität die Angiogenese fördert. Diese monoklonalen Antikörper hemmen auch das Wachstum von typischerweise weniger stark vaskularisierten Human-Kolonkarzinomen bei thymuslosen Mäusen und verringern die Anzahl der aus inokulierten zellen entstehenden Tumore.

Feste Tumore können mit Tyrosinkinaseinhibitoren behandelt werden, da diese Tumore für die Bildung der zur Unterstützung ihres Wachstums erforderlichen Blutgefäße auf Anglogenese angewiesen sind. Zu diesen festen Tumoren zählen die Monozytenleukämie, Hirn-, Urogenital-, Lymphsystem-, Magen-, Kehlkopf- und Lungenkarzinom, darunter Lungenadenokarzinom und kleinzelliges Lungenkarzinom.

Zu weiteren Beispielen fester Tumore zählen Karzinome, bei denen eine Überexpression oder Aktivierung von Raf-aktivierenden Onkogenen (z.B. K-ras, erb-B) beobachtet wird. Zu diesen Karzinomen zählen Bauchspeicheldrüsen- und Brustkarzinom. Hemmstoffe dieser Tyrosinkinasen und/oder Raf-Kinasen eignen sich daher zur Vorbeugung und Behandlung von proliferativen Krankheiten, die durch diese Enzyme bedingt sind.

Die angiogene Aktivität des VEGF ist nicht auf Tumore beschränkt. Der VEGF ist auch für die bei diabetischer Retinopathie in bzw. in der Nähe der Retina produzierte angiogene Aktivität verantwortlich. Dieses Gefäßwachstum in der Retina führt zu geschwächter Sehkraft und schließlich zur Erblindung. Die VEGF-mRNA- und -protein-Spiegel im Auge, die zur Gefäßneubildung führen, werden durch Leiden wie Netzhautvenenokklusion beim Primaten sowie verringertem pO₂-Spiegel bei der Maus, weiter erhöht. Intraokular injizierte monoklonale Anti-VEGF-Antikörper, oder VEGF-Rezeptor-Immunkonjugate, hemmen sowohl im Primaten- als auch im Nagetiermodell die Gefäßneubildung im Auge. Unabhängig vom Grund der Induktion des VEGF bei der diabetischen Retinopathie des Menschen, eignet sich die Hemmung des Augen-VEGF zur Behandlung dieser Krankheit.

Die Expression eines VEGF-bindenden Konstrukts von Flk-1, Flt-1, dem zur Entfernung der zytoplasmatischen Tyrosinkinasedomänen, jedoch unter Beibehaltung eines Membranankers, verkürzten Maus-KDR-Rezeptorhomologs, in Viren stoppt praktisch das Wachstum eines transplantierbaren Glioblastoms bei der Maus, vermutlich aufgrund des dominant-negativen Mechanismus der Heterodimerbildung mit transmembranösen Endothelzellen-VEGF-Rezeptoren. Embryostammzellen, die in der Nacktmaus üblicherweise in Form von festen Tumoren wachsen, bilden bei Knock-out aller beider VEGF-Allele keine nachweisbaren Tumore. Aus diesen Daten gemeinsam geht die Rolle des VEGF beim Wachstum fester Tumore hervor. Die Hemmung von KDR bzw. Flt-1 ist an der pathologischen Angiogenese beteiligt, und Hemmstoffe dieser Rezeptoren eignen sich zur Behandlung von Krankheiten, bei denen Angiogenese einen Teil der Gesamtpathologie, z.B. Entzündung, diabetische Retina-Vaskularisierung sowie verschiedene Formen von Krebs, darstellt, da bekannt ist, dass das Tumorwachstum angiogeneseabhängig ist (Weidner et al., N. Engl. J. Med., 324, S. 1-8, 1991).

Die vorliegende Erfindung richtet sich auf Verbindungen, die VEGFR regulieren, modulieren oder hemmen können und deren Verwendung zur Vorbeugung und/oder Behandlung von Erkrankungen im Zusammenhang mit unregulierter oder gestörter VEGFR-Aktivität. Insbesondere lassen sich die erfindungsgemäßen Verbindungen deshalb bei der Behandlung gewisser Krebsformen einsetzen, sowie bei durch pathologische Angiogenese bedingten Erkrankungen wie diabetische Retinopathie oder Entzündungen.
Weiterhin können erfindungsgemäße Verbindungen zur Isolierung und zur Untersuchung der Aktivität oder Expression von VEGFR verwendet werden. Außerdem eigenen sie sich insbesondere zur Verwendung in diagnostischen Verfahren zu Erkrankungen im Zusammenhang mit unregulierter oder gestörter VEGFR-Aktivität.

Bei Angiopoieten 1 (Ang1), einem Liganden für die endothelspezifische Rezeptor-Tyrosinkinase TIE-2, handelt es sich um einen neuen angiogenen Faktor (Davis et al, Cell, 1996, 87:1161-1169; Partanen et al, Mol. Cell Biol., 12:1698-1707 (1992); US-Patent Nr. 5,521,073; 5,879,672; 5,877,020; und 6,030,831). Das Akronym TIE steht für "Tyrosinkinase mit Ig- und EGF-Homologiedomänen". TIE wird zur Identifizierung einer Klasse von Rezeptor-Tyrosinkinasen verwendet, die ausschließlich in Gefäßendothelzellen und frühen hämopoietischen Zellen exprimiert werden. TIE-Rezeptorkinasen sind typischerweise durch das Vorhandensein einer EGF-ähnlichen Domäne und einer Immunglobulin (IG)-ähnlichen Domäne charakterisiert, die aus extrazellulären Faltungseinheiten, die durch Disulfidbrückenbindungen zwischen den Ketten stabilisiert sind, besteht (Partanen et al., Curr. Topics Microbiol. Immunol., 1999, 237:159-172). Im Gegensatz zu VEGF, der seine Funktion während der frühen Stadien in der Gefäßentwicklung ausübt, wirken Ang1 und sein Rezeptor TIE-2 während der späteren Stadien in der Gefäßentwicklung, d.h. während der Gefäßumbildung (Umbildung bezieht sich auf die Bildung eines Gefäßlumens) und Reifung (Yancopoulos et al., Cell, 1998, 93:661-664; Peters, K.G., Circ. Res., 1998, 83(3):342-3; Suri et al., Cell 87, 1171-1180 (1996)).

Demzufolge würde man erwarten, dass eine Hemmung von TIE-2 die Umbildung und Reifung eines durch Angiogenese initiierten neuen Gefäßsystems und dadurch den Angiogeneseprozeß unterbrechen sollte. Weiterhin würde eine Hemmung an der Kinasedomäne-Bindungsstelle von VEGFR-2 die Phosphorylierung von Tyrosinresten blockieren und dazu dienen, die Initiation der Angiogenese zu unterbrechen. Daher darf man annehmen, dass die Hemmung von TIE-2 und/oder VEGFR-2 die Tumorangiogenese verhindern und dazu dienen sollte, das Tumor-wachstum zu verlangsamen oder vollständig zu beseitigen. Dementsprechend könnte man mit Inhibitoren von TIE-2 und/oder VEGFR-2 eine Behandlung von Krebs und anderen mit unangemessener Angiogenese einhergehenden Erkrankungen bereitstellen.

Die vorliegende Erfindung richtet sich auf Verbindungen, die TIE-2 regulieren, modulieren oder hemmen können und deren Verwendung zur Vorbeugung und/oder Behandlung von Erkrankungen im Zusammenhang mit unregulierter oder gestörter TIE-2-Aktivität. Insbesondere lassen sich die erfindungsgemäßen Verbindungen deshalb bei der Behandlung gewisser Krebsformen einsetzen, sowie bei durch pathologische Angiogenese bedingten Erkrankungen wie diabetische Retinopathie oder Entzündungen.

Weiterhin können erfindungsgemäße Verbindungen zur Isolierung und zur Untersuchung der Aktivität oder Expression von TIE-2 verwendet werden. Außerdem eigenen sie sich insbesondere zur Verwendung in diagnostischen Verfahren zu Erkrankungen im Zusammenhang mit unregulierter oder gestörter TIE-2-Aktivität.

Weiterhin können die erfindungsgemäßen Verbindungen verwendet werden, um bei gewissen existierenden Krebschemotherapien und - bestrahlungen additive oder synergistische Effekte bereitzustellen, und/oder können dazu verwendet werden, um die Wirksamkeit gewisser existierender Krebschemotherapien und -bestrahlungen wiederherzustellen.

Die vorliegende Erfindung betrifft weiterhin die Verbindungen als Inhibitoren von Raf-Kinasen. Protein-Phosphorylierung ist ein fundamentaler Prozess für die Regulation von Zellfunktionen. Die koordinierte Wirkung von sowohl Proteinkinasen als auch Phosphatasen kontrolliert die Phosphorylierungsgrade und folglich die Aktivität spezifischer Zielproteine. Eine der vorherrschenden Rollen der Protein-Phosphorylierung ist bei der Signaltransduktion, wenn extrazelluläre Signale amplifiziert und durch eine Kaskade von Protein-Phosphorylierungs- und Dephosphorylierungsereignissen, z. B. im p21^{ras}/raf-Weg propagiert werden.

Das p21^{ras}-Gen wurde als ein Onkogen der Harvey- und Kirsten-Ratten-Sarkom-Viren (H-Ras bzw. K-Ras) entdeckt. Beim Menschen wurden charakteristische Mutationen im zellulären Ras-Gen (c-Ras) mit vielen verschiedenen Krebstypen in Verbindung gebracht. Von diesen mutanten Allelen, die Ras konstitutiv aktiv machen, wurde gezeigt, dass sie Zellen, wie zum Beispiel die murine Zelllinie NIH 3T3, in Kultur transformieren.

Das p21^{ras}-Onkogen ist ein wichtiger Faktor bei der Entwicklung und Progression humaner solider Karzinome und ist bei 30 % aller humaner Karzinome mutiert (Bolton et al. (1994) Ann. Rep. Med. Chem., 29, 165-74; Bos. (1989) Cancer Res., 49, 4682-9). In seiner normalen, nicht mutierten Form ist das Ras-Protein ein Schlüsselelement der Signaltransduktionskaskade, die durch Wachstumsfaktor-Rezeptoren in fast allen Geweben gesteuert wird (Avruch et al. (1994) Trends Biochem. Sci., 19, 279-83).

Biochemisch ist Ras ein Guanin-Nukleotid-bindendes Protein, und der Zyklus zwischen einer GTP-gebundenen aktivierten und einer GDPgebundenen ruhenden Form wird von Ras-endogener GTPase-Aktivität und anderen Regulatorproteinen strikt kontrolliert. Das Ras-Genprodukt bindet an Guanintriphosphat (GTP) und Guanindiphosphat (GDP) und hydrolysiert GTP zu GDP. Ras ist im GTP-gebundenen Zustand aktiv. In den Ras-Mutanten in Krebszellen ist die endogene GTPase-Aktivität abgeschwächt, und folglich gibt das Protein konstitutive Wachstumssignale an "Downstream"-Effektoren, wie zum Beispiel an das Enzym Raf-Kinase ab. Dies führt zum krebsartigen Wachstum der Zellen, die diese Mutanten tragen (Magnuson et al. (1994) Semin. Cancer Biol., 5, 247-53). Das Ras-Proto-Onkogen benötigt ein funktionell intaktes C-Raf-1-Proto-Onkogen, um in höheren Eukaryoten durch Rezeptor- und Nicht-Rezeptor-Tyrosin-Kinasen initiierte Wachstums- und Differenzierungssignale zu transduzieren.

Aktiviertes Ras ist für die Aktivierung des C-Raf-1-Proto-Onkogens notwendig, die biochemischen Schritte, durch die Ras die Raf-1-Protein-(Ser/Thr)-Kinase aktiviert, sind jedoch inzwischen gut charakterisiert. Es wurde gezeigt, dass das Inhibieren des Effekts von aktivem Ras durch Inhibition des Raf-Kinase-Signalwegs mittels Verabreichung von deaktivierenden Antikörpern gegen Raf-Kinase oder mittels Koexpression dominanter negativer Raf-Kinase oder dominanter negativer MEK (MAPKK), dem Substrat der Raf-Kinase, zur Reversion transformierter Zellen und zum normalen Wachstumsphänotyp führt (siehe: Daum et al. (1994) Trends Biochem. Sci., 19, 474-80; Fridman et al. (1994) J Biol. Chem., 269, 30105-8; Kolch et al. (1991) Nature, 349, 426-28); Besprechung Weinstein-Oppenheimer et al. Pharm. & Therap. (2000), 88, 229-279).

Auf ähnliche Weise wurde die Inhibition von Raf-Kinase (durch Antisense-Oligodesoxynukleotide) in vitro und in vivo mit der Inhibition des Wachstums einer Reihe verschiedener humaner Tumortypen in Beziehung gebracht (Monia et al., Nat. Med. 1996, 2, 668-75).

Raf-Serin- und Threonin-spezifische Protein-Kinasen sind zytosolische Enzyme, die das Zellwachstum in einer Reihe verschiedener Zellsysteme stimulieren (Rapp, U.R., et al. (1988) in The Oncogene Handbook; T. Curran, E.P. Reddy und A. Skalka (Hrsg.) Elsevier Science Publishers; Niederlande, S. 213-253; Rapp, U.R., et al. (1988) Cold Spring Harbor Sym. Quant. Biol. 53:173-184; Rapp, U.R., et al. (1990) Inv Curr. Top. Microbiol. Immunol. Potter und Melchers (Hrsg.), Berlin, Springer-Verlag 166:129-139).

Drei Isozyme wurden charakterisiert:
C-Raf (Raf-1) (Bonner, T.I., et al. (1986) Nucleic Acids Res. 14:1009-1015). A-Raf (Beck, T.W., et al. (1987) Nucleic Acids Res. 15:595-609), und B-Raf (Qkawa, S., et al. (1998) Mol. Cell. Biol. 8:2651-2654; Sithanandam, G. et al. (1990) Oncogene:1775). Diese Enzyme unterscheiden sich durch ihre Expression in verschiedenen Geweben. Raf-1 wird in allen Organen und in allen Zelllinien, die untersucht wurden, exprimiert, und A- und B-Raf werden in Urogenital- bzw. Hirngeweben exprimiert (Storm, S.M. (1990) Oncogene 5:345-351).

Raf-Gene sind Proto-Onkogene: Sie können die maligne Transformation von Zellen initiieren, wenn sie in spezifisch veränderten Formen exprimiert werden. Genetische Veränderungen, die zu onkogener Aktivierung führen, erzeugen eine konstitutiv aktive Proteinkinase durch Entfernen oder Interferenz mit einer N-terminalen negativen Regulatordomäne des Proteins (Heidecker, G., et al. (1990) Mol. Cell. Biol. 10:2503-2512; Rapp, U.R., et al. (1987) in Oncogenes and Cancer; S. A. Aaronson, J. Bishop, T. Sugimura, M. Terada, K. Toyoshima und P. K. Vogt (Hrsg.) Japan Scientific Press, Tokyo). Mikroinjektion in NIH 3T3-Zellen von onkogen aktivierten, aber nicht Wildtyp-Versionen des mit Expressionsvektoren von Escherichia coli präparierten Raf-Proteins führt zu morphologischer Transormation und stimuliert die DNA-Synthese (Rapp, U.R., et al. (1987) in Oncogenes and Cancer; S. A. Aaronson, J. Bishop, T. Sugimura, M. Terada, K. Toyoshima, und P. K. Vogt (Hrsg.) Japan Scientific Press, Tokyo; Smith, M. R., et al. (1990) Mol. Cell. Biol. 10:3828-3833).

Folglich ist aktiviertes Raf-1 ein intrazellulärer Aktivator des Zeilwachstums. Raf-1-Protein-Serin-Kinase ist ein Kandidat für den "Downstream"-Effektor der Mitogen-Signaltransduktion, da Raf-Onkogene der Apoptose begegnen, die aus einer Blockade zellulärer Ras-Aktivität aufgrund einer zellulären Mutation (Ras-revertante Zellen) oder Mikroinjektion von Anti-Ras-Antikörpern resultiert (Rapp, U.R., et al. (1988) in The Oncogene Handbook, T. Curran, E.P. Reddy und A. Skalka (Hrsg.), Elsevier Science Publishers; Niederlande, S. 213-253; Smith, M.R., et al. (1986) Nature (London) 320:540-543).

Die C-Raf-Funktion ist für die Transformation durch eine Reihe verschiedener Membran-gebundener Onkogene und für die Wachstumsstimulation durch in Seren enthaltenen Mitogene erforderlich (Smith, M.R., et al. (1986) Nature (London) 320:540-543). Raf-1-Protein-Serin-Kinase-Aktivität wird durch Mitogene über die Phosphorylierung reguliert (Morrison, D.K., et al. (1989) Cell 58:648-657), welche auch die subzelluläre Verteilung bewirkt (Olah, Z., et al. (1991) Exp. Brain Res. 84:403; Rapp, U.R., et al. (1988) Cold Spring Harbor Sym. Quant. Biol. 53:173-184. Zu Raf-1-aktivierenden Wachstumsfaktoren zählen der aus Thrombozyten stammende Wachstumsfaktor (PDGF) (Morrison, D.K., et al. (1988) Proc. Natl. Acad. Sci. USA 85:8855-8859), der Kolonien-stimulierende Faktor (Baccarini, M., et al. (1990) EMBO J. 9:3649-3657), Insulin (Blackshear, P.J., et al. (1990) J. Biol. Chem. 265:12115-12118), der epidermale Wachstumsfaktor (EGF) (Morrison, R.K., et al. (1988) Proc. Natl. Acad. Sci. USA 85:8855-8859), Interleukin-2 (Turner, B.C., et al. (1991) Proc. Natl. Acad. Sci. USA 88:1227) und Interleukin-3 und der Granulozyten-Makrophagen-Kolonien- stimulierende Faktor (Carroll, M.P., et al. (1990) J. Biol. Chem. 265:19812-19817).

Nach der Mitogen-Behandlung von Zellen transloziert die transient aktivierte Raf-1-Protein-Serin-Kinase in den perinukleären Bereich und den Nukleus (Olah, Z., et al. (1991) Exp. Brain Res. 84:403; Rapp, U.R., et al. (1988) Cold Spring Habor Sym. Quant. Biol. 53:173-184). Zellen, die aktiviertes Raf enthalten, sind in ihrem Genexpressionsmuster verändert (Heidecker, G., et al. (1989) in Genes and signal transduction in multistage carcinogenesis, N. Colburn (Hrsg.), Marcel Dekker, Inc., New York, S. 339-374) und Raf-oncogenes activate transcription from Ap-1/PEA3-dependent promotors in transient transfection assays (Jamal, S., et al. (1990) Science 344:463-466; Kaibuchi, K., et al. (1989) J. Biol. Chem. 264:20855-20858; Wasylyk, C., et al. (1989) Mol. Cell. Biol. 9:2247-2250).

Es gibt mindestens zwei unabhängige Wege für die Raf-1-Aktivierung durch extrazelluläre Mitogene: Einen, der Proteinkinase C (KC) beinhaltet, und einen zweiten, der durch Protein-Tyrosin-Kinasen initiiert wird (Blackshear, P.J., et al. (1990) J. Biol. Chem. 265:12131-12134; Kovacina, K.S., et al. (1990) J. Biol. Chem. 265:12115-12118; Morrison, D.K., et al. (1988) Proc. Natl. Acad. Sci. USA 85:8855-8859; Siegel, J.N., et al. (1990) J. Biol. Chem. 265:18472-18480; Turner, B.C., et al. (1991) Proc. Natl. Acad. Sci. USA 88:1227). In jedem Fall beinhaltet die Aktivierung eine Raf-1-Protein-Phosphorylierung. Die Raf-1-Phosphorylierung kann eine Folge einer Kinase-Kaskade sein, die durch Autophosphorylierung amplifiziert wird, oder sie kann vollkommen durch eine Autophosphorylierung hervorgerufen werden, die durch Bindung eines potentiellen Aktivierungsliganden an die Raf-1-Regulatordomäne, analog zur PKC-Aktivierung durch Diacylglycerol initiiert wird (Nishizuka, Y. (1986) Science 233:305-312).

Die vorliegende Erfindung richtet sich auf Verbindungen, die Raf-Kinasen regulieren, modulieren oder hemmen können und deren Verwendung zur Vorbeugung und/oder Behandlung von Erkrankungen im Zusammenhang mit unregulierter oder gestörter Aktivität von Raf-Kinasen. Insbesondere lassen sich die erfindungsgemäßen Verbindungen deshalb bei der Behandlung gewisser Krebsformen einsetzen. Wie oben bereits erwähnt, können die erfindungsgemäßen Verbindungen verwendet werden, um bei gewissen existierenden Krebschemotherapien und -bestrahlungen additive oder synergistische Effekte bereitzustellen, und/oder können dazu verwendet werden, um die Wirksamkeit gewisser existierender Krebschemotherapien und -bestrahlungen wiederherzustellen.

Weiterhin können erfindungsgemäße Verbindungen zur Isolierung und zur Untersuchung der Aktivität oder Expression von Raf-Kinasen verwendet werden. Außerdem eigenen sie sich insbesondere zur Verwendung in diagnostischen Verfahren zu Erkrankungen im Zusammenhang mit unregulierter oder gestörter Raf-Kinasen-Aktivität.

Einer der Hauptmechanismen, durch den die Zellregulation bewirkt wird, ist die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Protein-kinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine große Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279). Verschiedene Möglichkeiten zur Hemmung, Regulation und Modulation von Kinasen umfassen beispielsweise die Bereitstellung von Antikörpern, antisense-Ribozymen und Inhibitoren. In der Onkologieforschung sind insbesondere Tyrosinkinasen vielversprechende Targets. So sind zahlreiche synthetische kleine Moleküle als Tyrosinkinase-Inhibitoren zur Behandlung von Krebs in der klinischen Entwicklung z.B. Iressa^{®} oder Gleevec^{®}. Allerdings sind hier noch zahlreiche Probleme zu lösen, wie Nebenwirkungen, Dosierung, Resistenz des Tumors, Tumorspezifität und Patientenauswahl.

In der WO 02/44156 sind Benzimidazol-Derivate als TIE-2 und/oder VEGFR2-Inhibitoren beschrieben. In der WO 99/32436, WO 02/062763 WO 99/32455, WO 00/42012 und der WO 02/085857 sind Harnstoffderivate als Raf-Kinase-Inhibitoren offenbart.

Der Erfindung lag die Aufgabe zugrunde, neue Verbindungen mit wertvollen Eigenschaften aufzufinden, insbesondere solche, die zur Herstellung von Arzneimitteln verwendet werden können.

Die Identifikation und Bereitstellung von kleinen Verbindungen, die die Signaltransduktion der Tyrosinkinasen und/oder Raf-Kinasen spezifisch hemmen, regulieren und/oder modulieren, ist daher wünschenswert und ein Ziel der vorliegenden Erfindung.

Es wurde gefunden, dass die erfindungsgemäßen Verbindungen und ihre Salze bei guter Verträglichkeit sehr wertvolle pharmakologische Eigenschaften besitzen.

Insbesondere wurde gefunden, dass die erfindungsgemäßen Verbindungen überraschenderweise wirksame Kinase-Inhibitoren darstellen.

So zeigen sie eine Tyrosinkinasen-inhibierende Wirkung, insbesondere zeigen sie eine TIE-2 und/oder VEGFR-inhibierende Wirkung. Weiterhin stellen erfindungsgemäße wirksame Inhibitoren von Raf-Kinasen dar.

### Zusammenfassung der Erfindung

Gegenstand der Erfindung sind oben angegebenen Verbindungen der Formel I.

Für die gesamte Erfindung gilt, dass sämtliche Reste, die mehrfach auftreten, gleich oder verschieden sein können, d.h. unabhängig voneinander sind. Vor- und nachstehend haben die Reste bzw. Parameter die bei der Formel I angegebenen Bedeutungen, falls nicht ausdrücklich etwas anderes angegeben ist. Dementsprechend sind Gegenstand der Erfindung insbesondere diejenigen Verbindungen der Formel I, in denen mindestens einer der genannten Reste eine der nachstehend angegebenen bevorzugten Bedeutungen hat.

Hal bedeutet Fluor, Chlor, Brom oder lod, insbesondere Fluor oder Chlor.

A bedeutet Alkyl, ist unverzweigt (linear), verzweigt oder cyclisch, und hat 1, 2, 3, 4, 5, 6, 7, 8, 9, 10, 11, 12, 13 oder 14 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2-der 2,2-Dimethylpropyl, 1-Ethylpropyl, Hexyl, 1-, 2-, 3- oder 4-Methylpentyl, 1,1-, 1,2-, 1,3-, 2,2-, 2,3-oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, lineares oder verzweigtes Heptyl, Octyl, Nonyl oder Decyl bedeutet.

A bedeutet ganz besonders bevorzugt Alkyl mit 1, 2, 3, 4, 5 oder 6 C-Atomen, worin eine oder zwei CH₂-Gruppen durch O- oder S-Atome und/oder durch -CH=CH-Gruppen und/oder auch 1-7 H-Atome durch F und/oder Cl ersetzt sein können, wie z.B. Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

Cycloalkyl bedeutet vorzugsweise Cyclopropyl, Cyclobutyl, Cylopentyl, Cyclohexyl oder Cycloheptyl.

OA ist vorzugsweise Methoxy, ferner auch Ethoxy, n-Propoxy, Isopropoxy, n-Butoxy, Isobutoxy, sek.-Butoxy oder tert.-Butoxy.

Ar bedeutet z.B. unsubstituiertes Phenyl, Naphthyl oder Biphenyl, weiterhin vorzugsweise z.B. durch A, Fluor, Chlor, Brom, lod, Hydroxy, Methoxy, Ethoxy, Propoxy, Butoxy, Pentyloxy, Hexyloxy, Nitro, Cyan, Formyl, Acetyl, Propionyl, Trifluormethyl, Amino, Methylamino, Ethylamino, Dimethylamino, Diethylamino, Benzyloxy, Sulfonamido, Methylsulfonamido, Ethylsulfonamido, Propylsulfonamido, Butylsulfonamido, Dimethylsulfonamido, Phenylsulfonamido, Carboxy, Methoxycarbonyl, Ethoxycarbonyl, Aminocarbonyl mono-, di- oder trisubstituiertes Phenyl, Naphthyl oder Biphenyl.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m-oder p-Aminophenyl, o-, m- oder p-(N-Methylamino)-phenyl, o-, m- oder p-(N-Methylaminocarbonyl)-phenyl, o-, m- oder p-Acetamidophenyl, o-, m-oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m-oder p-Bromphenyl, o-, m- oder p- Chlorphenyl, o-, m- oder p-(Methylsulfonamido)-phenyl, o-, m- oder p-(Methylsulfonyl)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitropheriyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-di-methylamino- oder 3-Nitro-4-N,N-dimethylaminophenyl, 2,3-Diamino-phenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-Iodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Het bedeutet vorzugsweise unsubstituiertes oder ein-, zwei- oder dreifach z.B. durch Carbonylsauerstoff, F, Cl, Br, Methyl, Ethyl, Propyl, Phenyl, Benzyl, -CH₂-Cyclohexyl, Hydroxy, Methoxy, Ethoxy, Amino, Methylamino, Dimethylamino, Nitro, Cyan, Carboxy, Methoxycarbonyl, Aminocarbonyl, Methylaminocarbonyl, Dimethylaminocarbonyl, Acetamino,Ureido, Methylsulfonylamino, Formyl, Acetyl, Aminosulfonyl und/oder Methylsulfonyl substituiertes 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1-oder 5-Tetrazolyl, 1,2,3-Oxadiazol-4- oder -5-yl, 1,2,4-Oxadiazol-3- oder - 5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6-oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder-5-yl oder 2,1,3-Benzoxadiazol-5-yl.

Die heterocyclischen Reste können auch teilweise oder vollständig hydriert sein und bedeuten z.B. auch 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder-4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder-5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder-8-chinolyl, 1,2,3,4-Tetrahydro-1-,-2-,-3-, -4-, -5-, -6-, -7- oder-8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het bedeutet ferner z.B. 2-Oxo-piperidin-1-yl, 2-Oxo-pyrrolidin-1-yl, 2-Oxo-1*H*-pyridin-1-yl, 3-Oxo-morpholin-4-yl, 4-Oxo-1H-pyridin-1-yl, 2,6-Dioxo-piperidinl-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2H-pyridazin-2-yl, 2-Caprolactam-1-yl (= 2-Oxo-azepan-1-yl), 2-Hydroxy-6-oxo-piperazin-1-yl, 2-Methoxy-6-oxo-piperazin-1-yl oder 2-Aza-bicyclo[2.2.2]-octan-3-on-2-yl.

R¹ bedeutet vorzugsweise H, Hal, CF₃, NO₂, COOH oder COOR. R² bedeutet vorzugsweise H. R³ bedeutet vorzugsweise H, Hal oder CO-NHR. R bedeutet H,

Ⓨ bedeutet vorzugsweise Phenyl oder einen monocyclischen aromatischen Heterocyclus mit 1 bis 4 N-, O- und/oder S-Atomen, insbesondere bevorzugt bedeutet Ⓨ Phenyl, Furyl, Thienyl, Pyrrolyl, Imidazolyl, Pyridyl oder Pyrimidinyl, ganz besonders bevorzugt bedeutet Ⓨ Phenyl, Pyridyl oder Pyrimidinyl. X bedeutet vorzugsweise O, S, SO₂NH oder NH.

Die Bezeichnungen "Gruppe", "Rest", "Radikal", bzw. "Gruppen", "Reste", "Radikale" werden hier als Synonyme verwendet, so wie es in der Fachwelt üblich ist.

Die Bezeichnung "substituiert" bezieht sich vorzugsweise auf die Substitution mit den obengenannten Substituenten, wobei mehrere unterschiedliche Substitutionsgrade möglich sind, falls nicht anders angegeben.

Erfindungsgemäß sind auch alle physiologisch unbedenklichen Salze, Solvate und Stereoisomere dieser Verbindungen, einschließlich deren Mischungen in allen Verhältnissen.

Die Verbindungen der Formel I können ein oder mehrere chirale Zentren aufweisen. Sie können dementsprechend in verschiedenen enantiomeren Formen auftreten und in racemischer oder in optisch aktiver Form vorliegen. Gegenstand der Erfindung sind deshalb auch die optisch aktiven Formen (Stereoisomeren), die Enantiomeren, die Racemate, die Diastereomeren sowie Hydrate und Solvate dieser Verbindungen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wässrige oder alkoholische Lösungsmittelgemische wie z.B. Hexan/Isopropanol/ Acetonitril z.B. im Verhältnis 82:15:3.

Einige bevorzugte Gruppen von Verbindungen können durch die folgenden Teilformeln Ia bis le ausgedrückt werden, die der Formel I entsprechen und worin die nicht näher bezeichneten Reste die bei der Formel I angegebene Bedeutung haben, worin jedoch
- in Ia: R¹ Hal, NO₂, CF₃, COOH, COOR oder H bedeutet,
- in Ib: R² H bedeutet,
- in Ic: R³ H, Hal oder CO-NHR bedeutet,
- in Id: Y Phenyl, Furyl, Thienyl, Pyrrolyl, Imidazolyl, Pyridyl oder Pyrimidinyl bedeutet,
- in le: R¹ Hal, NO₂, CF₃, COOH, COOR oder H,
R² H,
R³ H, Hal oder CO-NHR,
Y Phenyl, Furyl, Thienyl, Pyrrolyl, Imidazolyl, Pyridyl oder Pyrimidinyl,
X O, S, SO₂NH oder NH,
n, p unabhängig voneinander 1, 2, 3 oder 4,
m 1
bedeuten,
sowie ihre pharmazeutisch unbedenklichen Salze, Solvate und Stereoisomere, einschließlich deren Mischungen in allen Verhältnissen.

Besonders bevorzugt sind die Verbindungen ausgewählt aus der Gruppe
a) Benzoxazol-2-yl-[4-(pyridin-4-yloxy)-phenyl]-amin,
b) Benzoxazol-2-yl-[4-(pyridin-4-ylsulfanyl)-phenyl]-amin,
c) N-Benzoxazol-2-yl-N'-pyridin-4-yl-benzol-1,4-diamin,
d) 2-[4-(Pyridin-4-ylsulfanyl)-phenylamino]-benzoxazol-5-carbonsäure,
e) 2-[4-(Pyridin-4-yloxy)-phenylamino]-benzoxazol-6-carbonsäure,
f) 2-[4-(Pyridin-4-ylsulfanyl)-phenylamino]-benzoxazol-6-carbonsäure,
g) 2-[4-(Pyridin-4-ylamino)-phenylamino]-benzoxazol-6-carbonsäuremethylester,
h) (5-Nitro-benzoxazol-2-yl)-[4-(pyridin-4-ylsulfanyl)-phenyl]-amin,
i) (5-Nitro-benzoxazol-2-yl)-[4-(pyridin-4-yloxy)-phenyl]-amin,
j) N-(5-Nitro-benzoxazol-2-yl)-N'-pyridin-4-yl-benzol-1,4-diamin,
k) (6-Nitro-benzoxazol-2-yl)-[4-(pyridin-4-yloxy)-phenyl]-amin,
l) (6-Nitro-benzoxazol-2-yl)-[4-(pyridin-4-ylsulfanyl)-phenyl]-amin,
m) N-(6-Nitro-benzoxazol-2-yl)-N'-pyridin-4-yl-benzol-1,4-diamin,
n) (5-Chlor-7-nitro-benzoxazol-2-yl)-[4-(pyridin-4-yloxy)-phenyl]-amin,
o) (5-Chlor-7-nitro-benzoxazol-2-yl)-[4-(pyridin-4-ylsulfanyl)-phenyl]-amin,
p) N-(5-Chlor-7-nitro-benzoxazol-2-yl)-N'-pyridin-4-yl-benzol-1,4-diamin,
q) (7-Brom-5-trifluormethyl-benzoxazol-2-yl)-[4-(pyridin-4-yloxy)-phenyl]-amin,
r) (7-Brom-5-trifluormethyl-benzoxazol-2-yl)-[4-(pyridin-4-ylsulfanyl)-phenyl]-amin,
s) (7-Brom-5-trifluormethyl-benzoxazol-2-yl)-[4-(4-fluor-phenylsulfanyl)-phenyl]-amin,
t) N-[4-(Brom-trifluormethyl-benzoxazol-2-ylamino)-phenyl]-4-fluor-benzolsulfonamid,
u) [4-(2-Amino-6-methyl-pyrimidin-4-yloxy)-phenyl]-(7-brom-5-trifluormethyl-benzoxazol-2-yl)-amin,
v) 4-[4-(Brom-trifluormethyl-benzoxazol-2-ylamino)-phenoxy]-pyridin-2-carbonsäuremethylamid,
w) 4-[4-(Brom-trifluormethyl-benzoxazol-2-ylamino)-phenylsulfanyl]-pyridin-2-carbonsäuremethylamid,
x) (7-Brom-5-trifluormethyl-benzoxazol-2-yl)-[4-(2,4-difluor-phenylsulfanyl)-phenyl]-amin,
sowie ihre pharmazeutisch unbedenklichen Salze, Solvate und Stereoisomere, einschließlich deren Mischungen in allen Verhältnissen.

Unter pharmazeutisch oder physiologisch unbedenklichen Derivaten versteht man z.B. Salze der erfindungsgemäßen Verbindungen, als auch sogenannte Prodrug-Verbindungen. Solche Derivate sind in dem Fachmann bekannt. Eine Übersicht zu physiologisch verträglichen Derivaten liefert Burger's Medicinal Chemistry And Drug Discovery, 5th Edition, Vol 1: Principles and Practice. Unter Prodrug-Verbindungen versteht man mit z.B. Alkyl- oder Acylgruppen, Zuckern oder Oligopeptiden abgewandelte Verbindungen der Formel I, die im Organismus rasch zu den wirksamen erfindungsgemäßen Verbindungen gespalten oder freigesetzt werden. Hierzu gehören auch bioabbaubare Polymerderivate der erfindungsgemäßen Verbindungen, wie dies z.B. in Int. J. Pharm. 115 (1995), 61-67 beschrieben ist.

Als Säureadditionssalze kommen anorganische oder organische Salze aller physiologisch oder pharmakologisch unbedenklichen Säuren in Frage, beispielsweise Halogenide, insbesondere Hydrochloride oder Hydrobromide, Lactate, Sulfate, Citrate, Tartrate, Maleate, Fumarate, Oxalate, Acetate, Phosphate, Methylsulfonate oder p-Toluolsulfonate.

Unter Solvaten der Verbindungen der Formel I werden Anlagerungen von inerten Lösungsmittelmolekülen an die Verbindungen der Formel I verstanden, die sich aufgrund ihrer gegenseitigen Anziehungskraft ausbilden. Solvate sind beispielsweise Hydrate, wie Monohydrate oder Dihydrate oder Alkoholate, d.h. Additionsverbindungen mit Alkoholen wie beispielsweise mit Methanol oder Ethanol.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder angestrebt wird.

Darüber hinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder Verhinderung von Nebenwirkungen oder auch die Verminderung des Fortschreitens einer Krankheit, eines Leidens oder einer Störung. Die Bezeichnung "therapeutisch wirksame Menge" umfasst auch die Mengen, die wirkungsvoll sind, die normale physiologische Funktion zu erhöhen.

Gegenstand der Erfindung sind auch Mischungen der erfindungsgemäßen Verbindungen der Formel I, z.B. Gemische zweier Diastereomere z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Verfahren zur Herstellung von Verbindungen der Formel I sowie ihrer physiologisch unbedenklichen Salze, Solvate und Stereoisomere, dadurch gekennzeichnet, dass man eine Verbindung der Formel II, 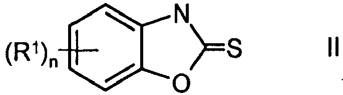 worin R¹ und n die oben angegebenen Bedeutungen haben, mit einer Verbindung der Formel III, 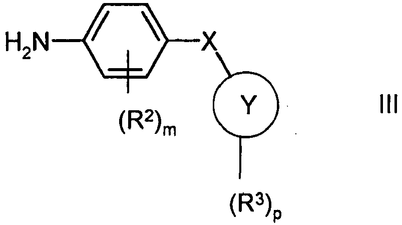 worin R², R³, X, Y, m und p die oben angegebenen Bedeutungen haben, umsetzt und/oder eine Base oder Säure der Formel I in eines ihrer Salze umwandelt.

Die Herstellung der Verbindungen der Formel I kann auch durch nachfolgendes Schema dargestellt werden.

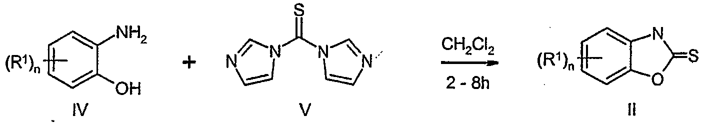

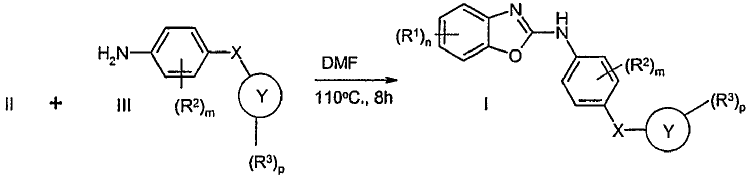

Besonders bevorzugt werden die Verbindungen der Formel IV und V in Gegenwart der Lösungsmittel Dichlormethan und Dimethylformamid bei Raumtemperatur über Nacht zu einer Verbindung der Formel II umgesetzt. Besonders bevorzugt werden die Verbindungen der Formel II und III in Gegenwart von Dimethylformamid bei 110°C über Nacht zu einer Verbindung der Formel I umgesetzt.

Es ist auch möglich die Reaktionen jeweils stufenweise durchzuführen.

Die Ausgangsverbindungen sind in der Regel bekannt. Sind sie neu, so können sie nach an sich bekannten Methoden hergestellt werden.

Die Ausgangsstoffe können, falls erwünscht, auch in situ gebildet werden, so dass man sie aus dem Reaktionsgemisch nicht isoliert, sondern sofort weiter zu den Verbindungen der Formel I umsetzt.

Die Ausgangsstoffe können in Abwesenheit eines Lösungsmittels in einem verschlossenen Reaktionsgefäß oder einem Autoklav zusammengeführt (verschmolzen) werden. Es ist jedoch auch möglich, die Ausgangsstoffe in Anwesenheit eines inerten Lösungsmittels reagieren zu lassen.

Die Umsetzung der Verbindungen der Formel II und II erfolgt nach Methoden, die dem Fachmann bekannt sind. Zunächst erfolgt die Reaktion in einem geeigneten Lösungsmittel, insbesondere in einem inerten Lösungsmittel.

Als inerte Lösungsmittel eignen sich z.B. Heptan, Hexan, Petrolether, Benzol, Toluol, Xylol, Trichlorethylen, 1,2- Dichlorethantetra-chlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether (bevorzugt für die Substitution am Indolstickstoff), Tetrahydrofuran (THF) oder Dioxan; Glykolether wie Ethylenglykolmonomethyl- oder monoethylether (Methylglykol oder Ethylglykol), Ethylenglykoldimethy-lether (Diglyme); Ketone wie Aceton oder Butanon; Amide wie Acetamid, Dimethylacetamid, N-Methylpyrrolidon (NMP) oder Dimethylformamid (DMF); Nitrile wie Acetonitril; Ester wie Ethylacetat, Carbonsäuren oder Säureanhydride, wie z. B. wie Essigsäure oder Acetanhydrid, Nitroverbindungen wie Nitromethan oder Nitrobenzol, gegebenenfalls auch Gemische der genannten Lösungsmittel untereinander oder Gemische mit Wasser. Besonders bevorzugt ist Dimethylformamid.

Die Reaktion kann auch in heterogener Phase ausgeführt werden, wobei vorzugsweise eine wässrige Phase und eine Benzol- oder Toluol-Phase verwendet werden. Hier kommt ein Phasentransfer-Katalysator zum Einsatz, wie beispielsweise Tetrabutylammoniumiodid und gegebenenfalls ein Acylierungskatalysator, wie beispielsweise Dimethylaminopyridin.

Die Menge des Lösungsmittels ist nicht kritisch, vorzugsweise können 10 g bis 500 g Lösungsmittel je g der umzusetzenden Verbindung der Formel I zugesetzt werden.

Geeignete Reaktionstemperaturen liegen bei Temperaturen von 10 bis 180°C, vorzugsweise bei 20 bis 150°C und ganz besonders bevorzugt bei 60 bis 120°C.

Bevorzugt wird bei einem Druck von 1 bis 200 bar gearbeitet, besonders bevorzugt bei Normaldruck.

Bevorzugt wird bei einem pH-Wert von 4 bis 10 gearbeitet.

Die Dauer der Umsetzung hängt von den gewählten Reaktionsbedingungen ab. In der Regel beträgt die Reaktionsdauer 0.5 Stunden bis 10 Tage, vorzugsweise 1 bis 24 Stunden, besonders bevorzugt 2 bis 12 Stunden.

Die Verbindungen der Formel I und auch die Ausgangsstoffe zu ihrer Herstellung werden im übrigen nach bekannten Methoden hergestellt, wie sie in der Literatur beschrieben sind (z. B. in Standardwerken wie Houben-Weyl, Methoden der organischen Chemie, Georg-Thieme-Verlag, Stuttgart) so z.B. unter Reaktionsbedingungen, die für die genannten Umsetzungen bekannt und geeignet sind. Dabei kann man auch von an sich bekannten, hier nicht näher beschriebenen Varianten Gebrauch machen.

Durch übliche Aufarbeitungsschritte wie z.B. Wasserzugabe zum Reaktionsgemisch und Extraktion können die Verbindungen der Formel I nach Entfernung des Lösungsmittels erhalten werden. Es kann vorteilhaft sein, zur weiteren Reinigung des Produktes eine Destillation oder Kristallisation anzuschließen.

Die Umsetzung von Verbindungen der Formel IV und V zu Verbindungen der Formel II erfolgt nach oben angegebenen Verfahren. Besonders bevorzugt werden als inerte Lösungsmittel Dichlormethan und Dimethylformamid verwendet. Bevorzugt wird bei Temperaturen von 10 bis 40 Grad, besonders bevorzugt bei Raumtemperatur und Normaldruck gearbeitet. Besonders bevorzugt beträgt die Reaktionsdauer 2 bis 12 Stunden.

Eine Säure der Formel I dann mit einer Base in das dazugehörige Additionssalz überführt werden, beispielsweise durch Umsetzung äquivalenter Mengen der Säure und der Base in einem inerten Lösungsmittel wie Ethanol und einschließendes Eindampfen. Für diese Umsetzung kommen insbesondere Basen in Frage, die physiologisch unbedenkliche Salze liefern. So kann die Säure der Formel I mit einer Base (z.B. Natrium- oder Kaliumhydroxid oder -carbonat) in das entsprechende Metall-, insbesondere Alkali- oder Erdalkalimetall- oder in das entsprechende Ammoniumsalz umgewandelt werden. Für diese Umsetzung kommen auch organische Basen in Frage, die physiologisch unbedenkliche Salze liefern, wie z.B. Ethanolamin.

Andererseits kann eine Base der Formel I mit einer Säure in das zugehörige Säureadditionssalz überführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wir Orthophosphorsäure, Sulfaminsäure, ferner organische Säuren, insbesondere aliphatische, alicyclische, araliphatische, aromatische oder heterocyclische, ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxysulfonsäure, Benzolsulfonsäure, p-Toluolsulfonsäure, Naphthalinmom- und disulfonsäuren oder Laurylschwefelsäure. Salze mit physiologisch nicht unbedenklichen Säuren, z.B. Pikrate, können zur Isolierung und/oder Aufreinigung der Verbindungen der Formel I verwendet werden.

Ein weiterer Gegenstand der Erfindung sind Arzneimittel, enthaltend wenigstens eine erfindungsgemäße Verbindung und/oder ihre physiologisch unbedenklichen Salze, Solvate und Stereoisomere, einschließlich deren Mischungen in allen Verhältnissen.
Weiterhin kann eine erfindungsgemäße pharmazeutische Zubereitung, weitere Träger- und/oder Hilfsstoffe sowie gegebenenfalls einen oder mehrere weitere Arzneimittelwirkstoffe enthalten.

Gegenstand der Erfindung ist ferner ein Verfahren zur Herstellung eines Arzneimittels, dadurch gekennzeichnet, dass man eine erfindungsgemäße Verbindung und/oder eines ihrer physiologisch unbedenklichen Salze, Solvate und Stereoisomere, einschließlich deren Mischungen in allen Verhältnissen zusammen mit einem festen, flüssigen oder halbflüssigen Träger- oder Hilfsstoff in eine geeignete Dosierungsform bringt.

Gegenstand der Erfindung ist auch ein Set (Kit) bestehend aus getrennten Packungen von
a) einer wirksamen Menge einer erfindungsgemäßen Verbindung und/oder ihrer physiologisch unbedenklichen Salze, Solvate und Stereoisomere, einschließlich deren Mischungen in allen Verhältnissen und
b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäßen Verbindung und/oder ihrer pharmazeutisch verwendbaren Solvate und Stereoisomere, einschließlich deren Mischungen in allen Verhältnissen, und einer wirksamen Menge eines weiteren Arzneimittelwirkstoffs gelöst oder in lyophylisierter Form vorliegt.

Arzneimittel können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche Arzneimitel mit einem der im pharmazeutischen Fachgebiet allgemein bekannten Verfahren herstellen.

Arzneimittel lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Arzneimittel können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepasste Arzneimittel können als separate Einheiten, wie z.B. Kapseln oder Tabletten; Pulver oder Granulate; Lösungen oder Suspensionen in wässrigen oder nichtwässrigen Flüssigkeiten; essbare Schäume oder Schaumspeisen; oder Öl-in-Wasser-Flüssigemulsionen oder Wasser-in-Öl-Flüssigemulsionen dargereicht werden.

So lässt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem essbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpresst wird, ein Schmiermittel und ein Sprengmittel zugegeben werden und das Ganze zu Tabletten verpresst wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch lässt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepresst wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengussformen zu verhindern. Das gefettete Gemisch wird dann zu Tabletten verpresst. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpresst werden. Eine durchsichtige oder undurchsichtige Schutzschicht, bestehend aus einer Versiegelung aus Schellack, einer Schicht aus Zucker oder Polymer-material und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so dass eine gegebene Quantität eine vorgegebene Menge der Verbindung enthält. Sirupe lassen sich herstellen, indem die Verbindung in einer wässrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung lässt sich auch so herstellen, dass die Freisetzung verlängert oder retardiert wird, wie beispielsweise durch Beschichtung oder Einbettung von partikulärem Material in Polymere, Wachs u.ä.

Die erfindungsgemäßen Verbindungen sowie Salze, Solvate und physiologisch funktionelle Derivate davon lassen sich auch in Form von Liposomenzuführsystemen, wie z.B. kleinen unilameliaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Verbindungen sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyano-acrylate und quervernetzte oder amphipatische Blockcopolymere von Hydrogelen, gekoppelt sein.

An die transdermale Verabreichung angepasste Arzneimittel können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepasste Arzneimittel können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer ÖI-in-Wasser-Cremebasis oder einer Wasser-in-Öl-Basis formuliert werden.

Zu den an die topische Applikation am Auge angepassten Arzneimittel gehören Augentropfen, wobei der Wirkstoff in einem geeigneten Träger, insbesondere einem wässrigen Lösungsmittel, gelöst oder suspendiert ist.

An die topische Applikation im Mund angepasste Arzneimittel umfassen Lutschtabletten, Pastillen und Mundspülmittel.

An die rektale Verabreichung angepasste Arzneimittel können in Form von Zäpfchen oder Einläufen dargereicht werden.

An die nasale Verabreichung angepasste Arzneimittel in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepasste Arzneimittel umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepasste Arzneimittel können als Pessare, Tampons, Cremes, Gele, Pasten, Schäume oder Sprayformulierungen dargereicht werden.

Zu den an die parenterale Verabreichung angepassten Arzneimittel gehören wässrige und nichtwässrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wässrige und nichtwässrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so dass nur die Zugabe der sterilen Trägerflüssigkeit, z.B. Wasser für injektionszwecke, unmittelbar vor Gebrauch erförderlich ist. Rezepturmäßig hergestellte Injektionslösungen und Suspensionen können aus sterilen Pulvern, Granulaten und Tabletten hergestellt werden.

Es versteht sich, dass die erfindungsgemäßen Arzneimittel neben den obigen besonders erwähnten Bestandteilen andere im Fachgebiet übliche Mittel mit Bezug auf die jeweilige Art der pharmazeutischen Formulierung enthalten können; so können beispielsweise für die orale Verabreichung geeignete Arzneimittel Geschmacksstoffe enthalten.

Eine therapeutisch wirksame Menge einer Verbindung der vorliegenden Erfindung hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so dass die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es lässt sich annehmen, dass ähnliche Dosierungen für die Behandlung der anderen, obenerwähnten Krankheitszustände geeignet sind.

### Verwendung

Die erfindungsgemäßen Verbindungen zeigen bevorzugt eine vorteilhafte biologische Aktivität, die in Enzym-Assays, wie in den Beispielen beschrieben, leicht nachweisbar ist. In derartigen auf Enzymen basierenden Assays zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Gegenstand der vorliegenden Erfindung sind erfindungsgemäße Verbindungen als Aktivatoren oder Inhibitoren, bevorzugt als Inhibitoren der hierin beschriebenen Signalwege. Besonders bevorzugter Gegenstand der Erfindung sind deshalb erfindungsgemäße Verbindungen als Aktivatoren und Inhibitoren von Kinasen, besonders bevorzugt als Inhibitoren von Tyrosinkinasen, insbesondere TIE-2 und/oder VEGFR, und/oder als Inhibitoren von Raf-Kinasen, insbesondere A-Raf, B-Raf und C-Raf-1.

Wie vorstehend besprochen, sind die durch die erfindungsgemäßen Verbindungen beeinflussten Signalwege für verschiedene Erkrankungen relevant. Dementsprechend sind die erfindungsgemäßen Verbindungen nützlich bei der Prophylaxe und/oder Behandlung von Erkrankungen, die von den genannten Signalwegen durch Interaktion mit einem oder mehreren der genannten Signalwege abhängig sind.

Ein weiterer Gegenstand der vorliegenden Erfindung ist deshalb die Verwendung von erfindungsgemäßen Verbindungen und/oder ihrer physiologisch unbedenklichen Salze, Solvate und Stereoisomere, einschließlich deren Mischungen in allen Verhältnissen zur Herstellung eines Medikaments zur Behandlung und/oder Prophylaxe von Krankheiten, insbesondere solcher Krankheiten, die durch Kinasen und/oder durch kinase-vermittelte Signaltransduktion verursacht, vermittelt und/oder propagiert werden. Bevorzugt sind hierbei Kinasen, ausgewählt aus der Gruppe der Tyrosinkinasen. Besonders bevorzugt handelt es sich bei den Tyrosinkinasen um TIE-2 oder VEGFR. Bevorzugt sind auch die Kinasen, ausgewählt aus der Gruppe der Raf-Kinasen. Besonders bevorzugt handelt es sich bei den Raf-Kinasen um A-Raf, B-Raf oder Raf-1.

Es wurde gefunden, dass die erfindungsgemäßen Verbindungen Inhibitoren des Enzyms Raf-Kinase sind. Da das Enzym ein "Downstream"- Effektor von p21^{ras} ist, erweisen sich die Inhibitoren in pharmazeutischen Zusammensetzungen für die human- oder veterinärmedizinische Anwendung als nützlich, wenn Inhibition des Raf-Kinase-Weges, z. B. bei der Behandlung von Tumoren und/oder durch Raf-Kinase vermitteltem krebsartigen Zellwachstum, angezeigt ist. Die Verbindungen sind insbesondere nützlich bei der Behandlung solider Karzinome bei Mensch und Tier, z. B. von murinem Krebs, da die Progression dieser Tumorarten von der Ras-Protein-Signaltransduktionskaskade abhängig ist und deshalb auf die Behandlung durch Unterbrechung der Kaskade, d. h. durch Inhibition der Raf-Kinase, anspricht. Dementsprechend wird die erfindungsgemäßen Verbindung oder ein pharmazeutisch unbedenkliches Salz davon für die Behandlung von Krankheiten verabreicht, die durch den Raf-Kinase-Weg vermittelt werden, besonders Krebs, einschließlich solider Karzinome, wie zum Beispiel Karzinome (z. B. der Lungen, des Pankreas, der Schilddrüse, der Harnblase oder des Kolons), myeloische Erkrankungen (z. B. myeloische Leukämie) oder Adenome (z. B. villöses Kolonadenom), pathologische Angiogenese und metastatische Zellmigration. Die Verbindungen sind ferner nützlich bei der Behandlung der Komplementaktivierungsabhängigen chronischen Entzündung (Niculescu et al. (2002) Immunol. Res., 24:191-199) und durch HIV-1 (Human Immunodeficiency Virus Typ 1) induzierte Immunschwäche (Popik et al. (1998) J Virol, 72: 6406-6413).

Außerdem eignen sich die vorliegenden Verbindungen als pharmazeutische Wirkstoffe für Säugetiere, insbesondere für den Menschen, bei der Behandlung von tyrosinkinasebedingten Krankheiten. Der Ausdruck "tyrosinkinasebedingte Krankheiten "bezieht sich auf pathologische Zustände, die von der Aktivität einer oder mehrerer Tyrosin-kinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubildung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Gewöhnlich werden die hier besprochenen Erkrankungen in zwei Gruppen eingeteilt, in hyperproliferative und nicht-hyperproliferative Erkrankungen. In diesem Zusammenhang werden Psoriasis, Arthritis, Entzündungen, Endometriose, Vernarbung, gutartige Prostatahyperplasie, immunologi-sche Krankheiten, Autoimmunkrankheiten und Immunschwächekrank-heiten als nicht-krebsartige Krankheiten angesehen, von denen Arthritis, Entzündung, immunologische Krankheiten, Autoimmunkrankheiten und Immunschwächekrankheiten gewöhnlich als nicht-hyperproliferative Erkrankungen angesehen werden.

In diesem Zusammenhang sind Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie als krebsartige Erkrankungen anzusehen, die alle gewöhnlich zur Gruppe der hyperproliferative Erkrankungen gezählt werden. Insbesondere krebsartiges Zellwachstum und insbesondere durch TIE-2, VEGFR- und Raf-Kinase direkt oder indirekt vermitteltes krebsartiges Zellwachstum ist eine Erkrankung, die ein Ziel der vorliegenden Erfindung darstellt.

Gegenstand der vorliegenden Erfindung ist deshalb die Verwendung von erfindungsgemäßen Verbindungen zur Herstellung eines Medikaments für die Behandlung und/oder Prophylaxe der genannten Erkrankungen sowie auch ein Verfahren zur Behandlung der genannten Erkrankungen, umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Empfänglichkeit einer bestimmten Zelle gegenüber der Behandlung mit den erfindungsgemäßen Verbindungen kann durch in vitro-Tests bestimmt werden. Typischerweise wird eine Kultur der Zelle mit einer erfindungsgemäßen Verbindung bei verschiedenen Konzentrationen für eine Zeitdauer inkubiert, die ausreicht, um den Wirkstoffen zu ermöglichen, Zelltod zu induzieren oder Migration zu inhibieren, gewöhnlich zwischen ungefähr einer Stunde und einer Woche. Zu in vitro-Tests können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der spezifischen Zellzahl und kann fortgesetzt werden, bis im Wesentlichen keine unerwünschten Zellen mehr im Körper nachgewiesen werden.

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).
Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J., unmittelbar vor der Veröffentlichung, Manuskript BJ20020786).

Es gibt viele mit einer Deregulation der Zellproliferation und des Zelltods (Apoptose) einhergehende Erkrankungen und Krankheitszustände. Die Erkrankungen und Krankheitszustände die durch erfindungsgemäße Verbindungen behandelt, verhindert oder gelindert werden können umfassen die nachfolgend aufgeführten Erkrankungen und Krankheitszustände, sind jedoch nicht darauf beschränkt. Die erfindungsgemäßen Verbindungen sind nützlich bei der Behandlung und/oder Prophylaxe einer Reihe verschiedener Erkrankungen und Krankheitszustände, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Die vorliegende Erfindung umfasst die Verwendung der erfindungsgemäßen Verbindungen zur Behandlung oder Vorbeugung von Krebs. Gegenstand der Erfindung ist insbesondere die Verwendung von erfindungsgemäßen Verbindungen und/oder ihrer physiologisch unbedenklichen Salze, Solvate und Stereoisomere, einschließlich deren Mischungen in allen Verhältnissen zur Herstellung eines Medikaments zur Behandlung und/oder Prophylaxe von festen Tumoren, wobei der feste Tumor besonders bevorzugt aus der Gruppe bestehend aus Gehirntumor, Tumor des Urogenitaltrakts, Tumor des lymphatischen Systems, Magentumor, Kehlkopftumor, Lungentumor ausgewählt ist. Bevorzugt können auch feste Tumore ausgewählt aus der Gruppe bestehend aus Monozytenleukämie, Lungenadenokarzinom, kleinzellige Lungenkarzinome, Bauchspeicheldrüsenkrebs, Glioblastome und Brustkarzinom mit Medikamenten enthaltend erfindungsgemäße Verbindungen behandelt werden.

Die erfindungsgemäßen Verbindungen können an Patienten zur Behandlung von Krebs verabreicht werden. Die vorliegenden Verbindungen hemmen die Tumorangiogenese und beeinflussen so das Wachstum von Tumoren (J. Rak et al. Cancer Research, 55:4575-4580, 1995). Die angiogenesehemmenden Eigenschaften der erfindungsgemäßen Verbindungen eignen sich auch zur Behandlung bestimmter Formen von Blindheit, die mit Retina-Gefäßneubildung in Zusammenhang stehen.

Gegenstand der Erfindung ist deshalb auch die Verwendung von erfindungsgemäßen Verbindungen und/oder ihrer physiologisch unbedenklichen Salze, Solvate und Stereoisomere, einschließlich deren Mischungen in allen Verhältnissen zur Herstellung eines Medikaments zur Behandlung und oder Prophylaxe von Krankheiten, die durch Angiogenese verursacht, vermittelt und/oder propagiert werden.

Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Gegenstand der Erfindung ist deshalb auch die Verwendung der erfindungsgemäßen Verbindungen zur Herstellung eines Medikaments zur Behandlung und/oder Prophylaxe der vorstehenden Erkrankungen.

Die Verwendung von erfindungsgemäßen Verbindungen und/oder ihrer physiologisch unbedenklichen Salze und Solvate zur Herstellung eines Medikaments zur Behandlung und/oder Prophylaxe von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.

Die erfindungsgemäßen Verbindungen eignen sich auch zur Behandlung bestimmter Knochen-Pathologien wie Osteosarkom, Osteoarthritis und Rachitis, die auch unter der Bezeichnung onkogene Osteomalazie bekannt ist (Hasegawa et al., Skeletal Radiol. 28, S.41-45, 1999; Gerber et al., Nature Medicine, Bd. 5, Nr. 6, S.623-628, Juni 1999). Da der VEGF durch den in reifen Osteoklasten exprimierten KDR/Flk-1 direkt die osteoklastische Knochenresorption fördert (FEBS Let. 473:161-164 (2000); Endocrinology, 141:1667 (2000)), eignen sich die vorliegenden Verbindungen auch zur Behandlung und Vorbeugung von Leiden, die mit Knochenresorption in Zusammenhang stehen, wie Osteoporose und Morbus Paget.

Ein weiterer Gegenstand der Erfindung ist deshalb die Verwendung erfindungsgemäßer Verbindungen und/oder ihrer physiologisch unbedenklichen Salze, Solvate und Stereoisomere, einschließlich deren Mischungen in allen Verhältnissen zur Herstellung eines Medikaments zur Behandlung von Knochen-Pathologien, ausgewählt aus der Gruppe bestehend aus Osteosarkom, Osteoarthritis und Rachitis.

Die Verbindungen können dadurch, dass sie zerebrale Ödeme, Gewebeschädigung und ischämiebedingte Reperfusionsverletzungen reduzieren, auch zur Verringerung oder Vorbeugung von Gewebeschäden, die nach zerebralen ischämischen Ereignissen wie Gehirnschlag auftreten, verwendet werden (Drug News Perspect 11:265-270 (1998); J. Clin. Invest. 104:1613-1620 (1999)).

Die erfindungsgemäßen Verbindungen eignen sich auch zur Herstellung eines Arzneimittels zur Behandlung und Prophylaxe von Krankheiten, die durch Raf-Kinasen verursacht, vermittelt und/oder propagiert werden, wobei die Raf-Kinase aus der Gruppe bestehend aus A-Raf, B-Raf und Raf-1 ausgewählt wird.

Bevorzugt ist die Verwendung zur Behandlung von Erkrankungen, vorzugsweise aus der Gruppe der hyperproliferativen und nicht-hyperproliferativen Erkrankungen.
Hierbei handelt es sich um Krebserkrankungen oder nicht-krebsartige Erkrankungen.

Gegenstand der Erfindung ist auch Verwendung erfindungsgemäßer Verbindungen und/oder ihrer physiologisch unbedenklichen Salze, Solvate und Stereoisomere, einschließlich deren Mischungen in allen Verhältnissen zur Herstellung eines Medikaments zur Behandlung von Krankheiten, ausgewählt aus der Gruppe der nicht-krebsartigen Erkrankungen bestehend aus Psoriasis, Arthritis, Entzündungen, Endometriose, Vernarbung, gutartiger Prostatahyperplasie, immunologischer Krankheiten, Autoimmunkrankheiten und Immunschwächekrankheiten.

Ein weiterer Gegenstand der Erfindung ist die Verwendung von erfindungsgemäßen Verbindungen und/oder ihrer physiologisch unbedenklichen Salze, Solvate und Stereoisomere, einschließlich deren Mischungen in allen Verhältnissen zur Herstellung eines Medikaments zur Behandlung von Krankheiten, ausgewählt aus der Gruppe der krebsartigen Erkrankungen bestehend aus Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischem Krebs, Schilddrüsenkrebs, Lymphom, chronischer Leukämie und akuter Leukämie.

Die erfindungsgemäßen Verbindungen können auch gemeinsam mit anderen gut bekannten Therapeutika, die aufgrund ihrer jeweiligen Eignung für das behandelte Leiden ausgewählt werden, verabreicht werden. So wären zum Beispiel bei Knochenleiden Kombinationen günstig, die antiresorptiv wirkende Bisphosphonate, wie Alendronat und Risedronat, Integrinblocker (wie sie weiter unten definiert werden), wie avβ3-Antagonisten, bei der Hormontherapie verwendetete konjugierte Östrogene wie Prempro®, Premarin® und Endometrion®; selektive Östrogenrezeptormodulatoren (SERMs) wie Raloxifen, Droloxifen, CP-336,156 (Pfizer) und Lasofoxifen, Kathepsin-K-Inhibitoren und ATP-Protonenpumpeninhibitoren enthalten.
Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferaseinhibitoren, HMG-CoA-Reduktase-Inhibitoren, HIV-Protease-Inhibitoren, Reverse-Transkriptase-Inhibitoren, Wachstumsfaktor-Inhibitoren sowie Angiogeneseinhibitoren. Die vorliegenden Verbindungen eignen sich insbesondere zur gemeinsamen Anwendung mit Radiotherapie. Die synergistischen Wirkungen der Hemmung des VEGF in Kombination mit Radiotherapie sind in der Fachwelt beschrieben worden (siehe WO 00/61186). "Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Inhibitoren, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylomithin, ILX23-7553, trans-N-(4'-Hydroxy-phenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Inhibitoren und Topoisomerase-Inhibitoren.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyl-daunorubicin (siehe WO 00/50032), was jedoch keine Einschränkung darstellen soll.
Zu den Mikrotubulin-Inhibitorenn zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Inhibitoren sind zum Beispiel Topotecan, Hycaptamin, Irinotecan, Rubitecan, 6-Ethoxypropionyl-3',4'-O-exo-benzylidenchartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNP11100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Amino-propylamino)-7,10-dihydroxy-2-(2-hydroxyethylaminomethyl)-6H-pyrazolo[4,5,1-de]acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryi)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diaza-tetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Inhibitorenn" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
- Massenspektrometrie (MS):: EI (Elektronenstoß-Ionisation) M⁺.
FAB (Fast Atom Bombardment) (M+H)⁺
ESI (Electrospray lonization) (M+H)⁺ (wenn nichts anderes angegeben)

### Beispiel 1:

Die in den Beispielen beschriebenen erfindungsgemäßen Verbindungen wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).
VEGF-Rezeptorkinase-Assay
Die VEGF-Rezeptorkinaseaktivität wird durch Einbau von radioaktiv markiertem Phosphat in 4:1 Polyglutaminsäure/Tyrosin-Substrat (pEY) bestimmt. Das phosphorylierte pEY-Produkt wird auf einer Filtermembran festgehalten, und der Einbau des radioaktiv markierten Phosphats wird durch Szintillationszählung quantitativ bestimmt.

### VEGF-Rezeptorkinase

Die intrazelluläre-Tyrosinkinase-Domänen des menschlichen KDR (Terman, B. I. et al. Oncogene (1991) Bd. 6, S. 1677-1683.) und Flt-1 (Shibuya, M. et al. Oncogene (1990) Bd. 5, S. 519-524) wurden als Glutathion-S-transferase (GST)-Genfusionsproteine kloniert. Dies geschah durch Klonieren der Zytoplasma-Domäne der KDR-Kinase als leserastergerechte Fusion am Carboxy-Terminus des GST-Gens. Die löslichen rekombinanten GST-Kinasedomäne-Fusionsproteine wurden in *Spodoptera frugiperda* (Sf21) Insektenzellen (Invitrogen) unter Verwendung eines Baculovirus-Expressionsvektors (pAcG2T, Pharmingen) exprimiert.

Lysepuffer: 50 mM Tris pH 7,4, 0,5 M NaCl, 5 mM DTT, 1 mM EDTA, 0,5% Triton X-100, 10% Glycerin, je 10 mg/ml Leupeptin, Pepstatin und Aprotinin sowie 1 mM Phenylmethylsulfonylfluorid (alle von Sigma).
Waschpuffer: 50 mM Tris pH 7,4, 0,5 M NaCl, 5 mM DTT, 1 mM EDTA, 0.05% Triton X-100, 10% Glycerin, je 10 mg/ml Leupeptin, Pepstatin und Aprotinin sowie 1 mM Phenylmethylsulfonylfluorid.
Dialysepuffer: 50 mM Tris pH 7,4, 0,5 M NaCl, 5 mM DTT, 1 mM EDTA, 0.05% Triton X-100, 50% Glycerin, je 10 mg/ml Leupeptin, Pepstatin und Aprotinin sowie 1 mM Phenylmethylsulfonylfluorid.
10× Reaktionspuffer: 200 mM Tris, pH 7,4, 1,0 M NaCl, 50 mM MnCl₂, 10 mM DTT und 5 mg/ml Rinderserumalbumin [bovine serum albumin = BSA] (Sigma).
Enzymverdünnuncispuffer: 50 mM Tris, pH 7,4, 0,1 M NaCl, 1 mM DTT, 10% Glycerin, 100 mg/ml BSA.
10× Substrat: 750 µg/ml Poly(glutaminsäure/Tyrosin; 4:1) (Sigma). Stopp-Lösung: 30% Trichloressigsäure, 0,2 M Natriumpyrophosphat (beide von Fisher).
Waschlösung: 15% Trichloressigsäure, 0,2 M Natriumpyrophosphat. Filterplatten: Millipore #MAFC NOB, GF/C 96-Well-Glasfaserplatte.

### Verfahren A - Proteinaufreinigung:

1. Die Sf21-Zellen werden mit dem rekombinanten Virus bei einer m.o.i. (Multiplizität der Infektion) von 5 ViruspartikeIn/Zelle infiziert und 48 Stunden lang bei 27°C gezüchtet.
2. Alle Schritte werden bei 4°C durchgeführt. Die infizierten Zellen werden durch Zentrifugieren bei 1000xg geerntet und 30 Minuten bei 4°C mit 1/10 Volumen Lysepuffer lysiert und anschließend 1 Stunde lang bei 100.000xg zentrifugiert. Der Überstand wird dann über eine mit Lysepuffer äquilibrierte Glutathion-Sepharose-Säure (Pharmacia) gegeben und mit 5 Volumina des gleichen Puffers und anschließend 5 Volumina Waschpuffer gewaschen. Das rekombinante GST-KDR-Protein wird mit Waschpuffer/10 mM reduziertem Glutathion (Sigma) eluiert und gegen Dialysepuffer dialysiert.

### Verfahren B -VEGF-Rezeptorkinase-Assav:

1. Assay mit 5 µl Hemmstoff oder Kontrolle in 50% DMSO versetzen.
2. Mit 35 µl Reaktionsmischung, die 5 µl 10× Reaktionspuffer, 5 µl 25 mM ATP/10 µCi[³³ P]ATP (Amersham) und 5 µl 10× Substrat enthält, versetzen.
3. Reaktion durch Zugabe von 10 µl KDR (25 nM) in Enzymverdünnungspuffer starten.
4. Mischen und 15 Minuten lang bei Raumtemperatur inkubieren.
5. Reaktion durch Zugabe von 50 µl Stopp-Lösung stoppen.
6. 15 Minuten lang bei 4°C inkubieren.
7. 90-µl-Aliquot auf Filterplatte überführen.
8. Absaugen und 3 Mal mit Waschlösung waschen.
9. 30 µl Szintillations-Cocktail zugeben, Platte verschließen und in einem Szintillations-Zähler Typ Wallac Microbeta zählen.

### Mitogenese-Assay an menschlichen Nabelschnurvenenendothelzellen:

Die Expression von VEGF-Rezeptoren, die mitogene Reaktionen auf den Wachstumsfaktor vermitteln, ist größtenteils auf Gefäßendothelzellen beschränkt. Kultivierte menschliche Nabelschnurvenenendothelzellen (HUVECs) proliferieren als Reaktion auf Behandlung mit VEGF und können als Assaysystem zur quantitativen Bestimmung der Auswirkungen von KDR-Kinaseinhibitorenn auf die Stimulation des VEGF verwendet werden. In dem beschriebenen Assay werden Einzelzellschichten von HUVECs im Ruhezustand 2 Stunden vor der Zugabe von VEGF oder "basic fibroblast growth factor" (bFGF) mit dem Konstituens oder der Testverbindung behandelt. Die mitogene Reaktion auf VEGF oder bFGF wird durch Messung des Einbaus von [³H]Thymidin in die Zell-DNA bestimmt.

### HUVECs

Als Primärkulturisolate tiefgefrorene HUVECs werden von Clonetics Corp bezogen. Die Zellen werden im Endothel-Wachstumsmedium (Endothelial Growth Medium = EGM; Clonetics) erhalten und in der 3. - 7. Passage für die Mitogenitätsassays verwendet.
Kulturplatten: NUNCLON 96-Well-Polystyrol-Gewebekulturplattten (NUNC #167008).
Assay-Medium: Nach Dulbecco modifiziertes Eagle-Medium mit 1 g/ml Glucose (DMEM mit niedrigem Glucosegehalt; Mediatech) plus 10% (v/v) fötales Rinderserum (Clonetics).
Testverbindungen: Mit den Arbeitsstammlösungen der Testverbindungen wird mit 100% Dimethylsulfoxid (DMSO) solange eine Reihenverdünnung durchgeführt, bis ihre Konzentrationen um das 400-fache höher als die gewünschte Endkonzentration sind. Die letzten Verdünnungen (Konzentration 1 ×) werden unmittelbar vor Zugabe zu den Zellen mit Assay-Medium hergestellt.
10× Wachstumsfaktoren: Lösungen des menschlichen VEGF 165 (500 ng/ml; R&D Systems) und bFGF (10 ng/ml; R&D Systems) werden mit Assay-Medium hergestellt.
10× [³H]-Thymidin: [Methyl-³H]-Thyrmidin (20 Ci/mmol; Dupont-NEN) wird mit DMEM-Medium mit niedrigem Glucosegehalt auf 80 µCi/ml verdünnt.
Zellwaschmedium: Hank's balanced salt solution (Mediatech) mit 1 mg/ml Rinderserumalbumin (Boehringer-Mannheim).
Zell-Lyse-Lösung: 1 N NaOH, 2% (w/v) Na₂CO₃.

### Verfahren 1

In EGM gehaltene HUVEC-Einzelzellschichten werden durch Trypsinbehandlung geerntet und in einer Dichte von 4000 Zellen pro 100 µl Assay-Medium pro Näpfchen in 96-Well-Platten überimpft. Das Wachstum der Zellen wird 24 Stunden bei 37°C in einer 5% CO₂ enthaltenden feuchten Atmosphäre gestoppt.

### Verfahren 2

Das Wachstumsstoppmedium wird durch 100 µl Assay-Medium ersetzt, das entweder das Konstituens (0,25% [v/v] DMSO) oder die erwünschte Endkonzentration der Testverbindung enthält. Alle Bestimmungen werden in dreifacher Wiederholung durchgeführt. Die Zellen werden dann 2 Stunden bei 37°C/5% CO₂ inkubiert, so dass die Testverbindungen in die Zellen eindringen können.

### Verfahren 3

Nach 2-stündiger Vorbehandlung werden die Zellen durch Zugabe von 10 µl Assay-Medium, 10x VEGF-Lösung oder 10x bFGF-Lösung pro Näpfchen stimuliert. Die Zellen werden dann bei 37°C/5% CO₂ inkubiert.

### Verfahren 4

Nach 24 Stunden in Anwesenheit der Wachstumsfaktoren wird mit 10× [³H]-Thymidin (10 µl/well) versetzt.

### Verfahren 5

Drei Tage nach dem Versetzen mit [³H]-Thymidin wird das Medium abgesaugt und die Zellen werden zweimal mit Zellwaschmedium gewaschen (400 µl/well, anschließend 200 µl/well). Die gewaschenen, adhärenten Zellen werden dann durch Zugabe von Zell-Lyse-Lösung (100 µl/well) und 30-minutiger Erwärmen auf 37°C solubilisiert. Die Zell-Lysate werden in 7-ml-Szintillationsrährchen aus Glas, die 150 µl Wasser enthalten, überführt. Man versetzt mit dem Szintillations-Cocktail (5 ml/Röhrchen), und die mit den Zellen assoziierte Radioaktivität wird flüssigkeitsszintillationsspektroskopisch bestimmt.
Gemäß diesen Assays stellen die Verbindungen der Formel I VEGF-Inhibitoren dar und eignen sich daher zur Hemmung der Angiogenese, wie bei der Behandlung von Augenkrankheiten, z.B. diabetischer Retinopathie, und zur Behandlung von Karzinomen, z.B. festen Tumoren. Die vorliegenden Verbindungen hemmen die VEGF-stimulierte Mitogenese von kultivierten menschlichen Gefäßendothelzellen mit HK50-Werten von 0,01-5,0 µM. Diese Verbindungen sind im Vergleich zu verwandten Tyrosinkinasen (z.B. FGFR1 sowie Src-Familie; zur Beziehung zwischen Src-Kinasen und VEGFR-Kinasen siehe Eliceiri et al., Molecular Cell, Bd. 4, S.915-924, Dezember 1999) auch selektiv.

Die TIE-2-Tests können z.B. analog der in WO 02/44156 angegebenen Methoden durchgeführt werden.

Der Assay bestimmt die inhibierende Aktivität der zu testenden Substanzen bei der Phosphorylierung des Substrats Poly(Glu, Tyr) durch Tie-2-Kinase in Gegenwart von radioaktivem ³³P-ATP. Das phosphorylierte Substrat bindet während der Inkubationszeit an die Oberfläche einer "flashplate"-Mikrotiterplatte. Nach Entfernen der Reaktionsmischung wird mehrmals gewaschen und anschließend die Radioaktivität an der Oberfläche der Mikrotiterplatte gemessen. Ein inhibierender Effekt der zu messenden Substanzen hat eine geringere Radioaktivität, verglichen mit einer ungestörten enzymatischen Reaktion, zur Folge.

### Beispiel 2: Herstellung von (5-Chlor-7-nitro-benzoxazol-2-yl)-[4-(pyridin-4-ylsulfanyl)-phenyl]-amin

Die Herstellung erfolgt analog nachstehendem Schema 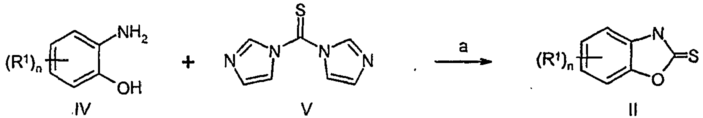 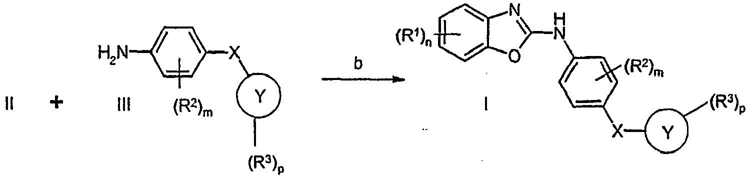
a 2 g 2-Amino-4-chlor-6-nitro-phenol und 0.92 g Di-imidazol-1-yl-methanthion werden in 35 ml Dichlormethan und 15 ml Dimethylformamid gelöst und über Nacht bei Raumtemperatur gerührt. Die Lösung wird mit 1N HCl extrahiert. Die organische Phase wird mit MgSO₄ getrocknet abfiltriert und im Vakuum zum Rückstand eingeengt. Man erhält 1.56 g 5-Chlor-7-nitro-3*H-*benzoxazol-2-thion.
b 100 mg 5-Chlor-7-nitro-3*H*-benzoxazol-2-thion. und 88 mg 4-(Pyridin-4-ylsulfanyl)-phenylamin werden in 3 ml Dimethylformamid gelöst und bei 110°C über Nacht gerührt. Man dampft im Vakuum zum Rückstand ein, welcher mit Hilfe der präparativen HPLC gereinigt wird. Man erhält 40 mg (5-Chlor-7-nitro-benzoxazol-2-yl)-[4-(pyridin-4-ylsulfanyl)-phenyl]-amin, einer amorphen Festsubstanz; Retentionszeit: 3.68 Minuten, EI-MS (M+H)⁺ 399.

### Methode zur Bestimmung der Retentionszeit:

| | |
|---|---|
| Säule: | Chromolith SpeedROD, 50 x 4.6mm² von Merck |
| Fließmittel: | A: H₂O, 0,1%TFA |
| | B: Acetonitril, 0,08% TFA |
| Wellenlänge: | 220 nm |
| Gradient: | 5.0 min, t = 0 min, A:B = 95: 5, t = 4.4 min: A:B = 25:75, t = 4.5 min bis t = 5.0 min: A:B = 0:100 |
| Fluß: | 3.00 ml/min |

### Analog erhält man nachstehende Verbindungen

| **Verbindung** | **Mol.-Gewicht** EI-MS (M+H)⁺ | **Retentionszeit** (min) |
|---|---|---|
| 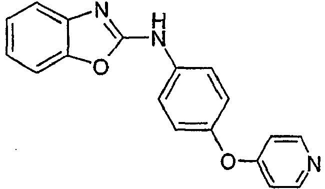 | | |
| Benzoxazol-2-yl-[4-(pyridin-4-yloxy)-phenyl]-amin | 3,03E+02 | 2,93 |
| 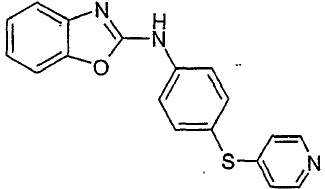 | | |
| Benzoxazol-2-yl-[4-(pyridin-4-ylsulfanyl)-phenyl]-amin | 3,19E+02 | 3,15 |
| 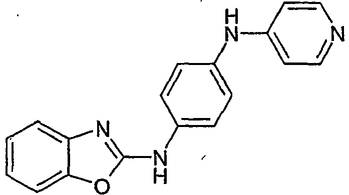 | | |
| N-Benzoxazol-2-yl-N'-pyridin-4-yl-benzol-1,4-diamin | 3,02E+02 | 2,75 |
| 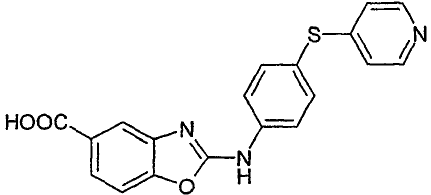 | | |
| 2-[4-(Pyridin-4-ylsulfanyl)-phenylamino]-benzoxazol-5-carbonsäure | 3,63E+02 | 2,72 |
| 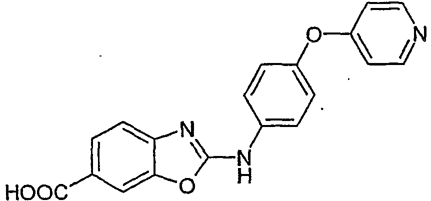 | 3,47E+02 | 2,4 |
| 2-[4-(Pyridin-4-yloxy)-phenylamino]-benzoxazol-6-carbonsäure | | |
| 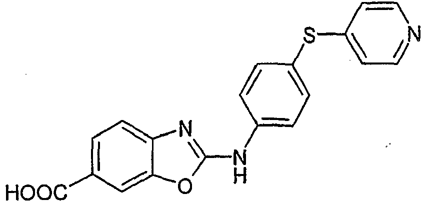 | | |
| 2-[4-(Pyridin-4-ylsulfanyl)-phenylamino]-benzoxazol-6-carbonsäure | 3,63E+02 | 2,69 |
| 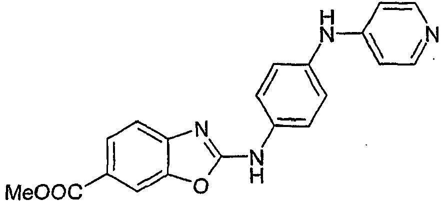 | | |
| 2-[4-(Pyridin-4-ylamino)-phenylamino]-benzoxazol-6-carbonsäuremethylester | 3,60E+02 | 2,83 |
| 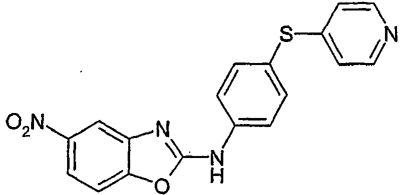 | | |
| (5-Nitro-benzoxazol-2-yl)-[4-(pyridin-4-ylsulfanyl)-phenyl]-amin | 3,64E+02 | 3,28 |
| 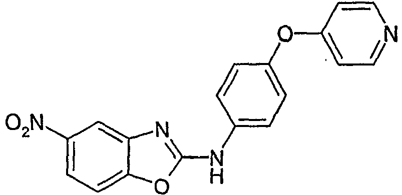 | | |
| (5-Nitro-benzoxazol-2-yl)-[4-(pyridin-4-yloxy)-phenyl]-amin | 3,48E+02 | 3,12 |
| 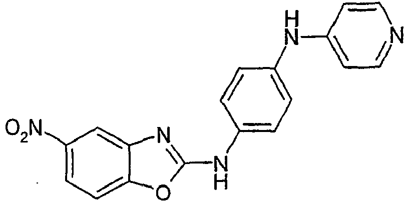 | | |
| N-(5-Nitro-benzoxazol-2-yl)-N'-pyridin-4-yl-benzol-1,4-diamin | 3,47E+02 | 3,09 |
| 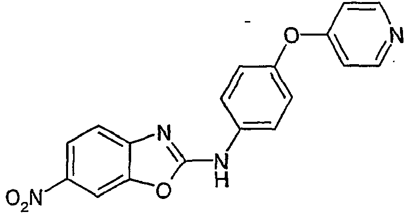 | | |
| (6-Nitro-benzoxazol-2-yl)-[4-(pyridin-4-yloxy)-phenyl]-amin | 3,48E+02 | 3,07 |
| 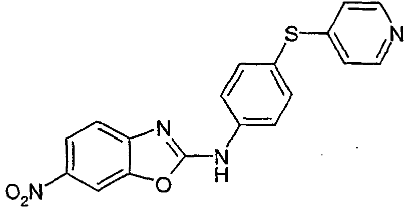 | | |
| (6-Nitro-benzoxazol-2-yl)-[4-(pyridin-4-yfsuffanyl)-phenyl]-amin | 3,64E+02 | 3,2 |
| 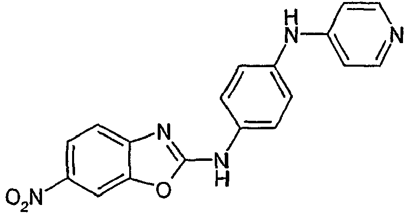 | | |
| N-(6-Nitro-benzoxazol-2-yl)-N'-pyridin-4-yl-benzol-1,4-diamin | 3,47E+02 | 2,93 |
| 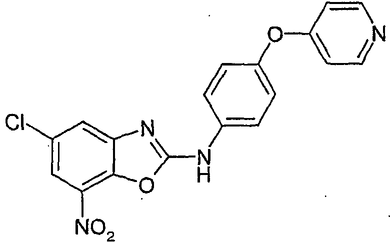 | | |
| (5-Chlor-7-nitro-benzoxazol-2-yl)-[4-(pyridin-4-yloxy)-phenyl]-amin | 3,83E+02 | 3,44 |
| 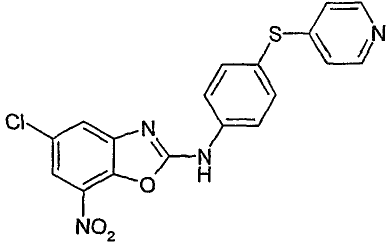 | | |
| (5-Chlor-7-nitro-benzoxazol-2-yl)-[4-(pyridin-4-ylsulfanyl)-phenyl]-amin | 3,99E+02 | 3,68 |
| 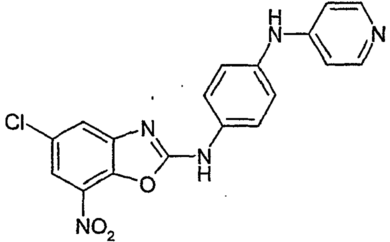 | | |
| N-(5-Chlor-7-nitro-benzoxazol-2-yl)-N'-pyridin-4-yl-benzol-1,4-diamin | 3,82E+02 | 3,49 |
| 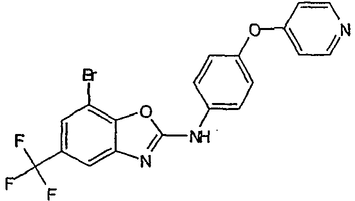 | | |
| (7-Brom-5-trifluormethyl-benzoxazol-2-yl)-[4-(pyridin-4-yloxy)-phenyl]-amin | 4,50E+02 | 3,84 |
| 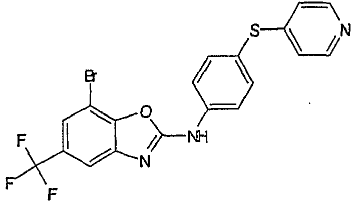 | | |
| (7-Brom-5-trifluormethyl-benzoxazol-2-yl)-[4-(pyridin-4-ylsulfanyl)-phenyl]-amin | 4,66E+02 | 4,05 |
| 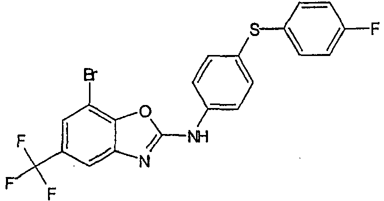 | | |
| (7-Brom-5-trifluormethyl-benzoxazol-2-yl)-[4-(4-fluor-phenylsulsulfanyl)-phenyl]-amin | 483E+02 | 5,65 |
| 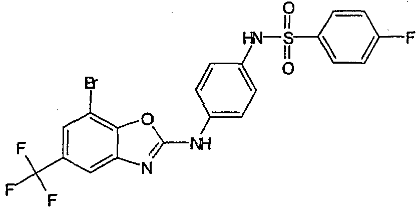 | | |
| N-[4-(Brom-trifluormethyl-benzoxazol-2-ylamino)-phenyl]-4-fluor-benzolsulfonamid | 5,30E+02 | 5,01 |
| 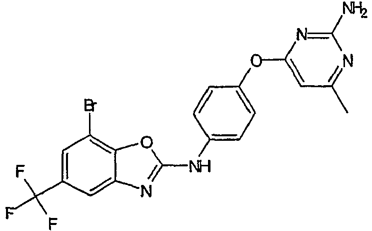 | | |
| [4-(2-Amino-6-methyl-pyrimidin-4-yloxy)-phenyl]-(7-brom-5-trifluormethyl-benzoxazol-2-yl)-amin | 4,80E+02 | 4,08 |
| 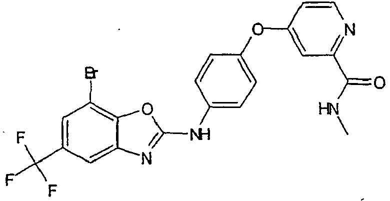 | | |
| 4-[4-(Brom-trifluormethyl-benzoxazol-2-ylamino)-phenoxy]-pyridin-2-carbonsäuremethylamid | 5,07E+02 | 4,72 |
| 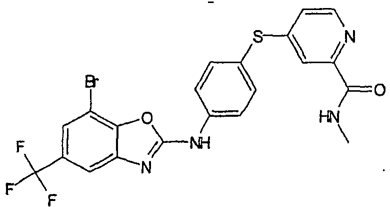 | | |
| 4-[4-(Brom-trifluormethyl-benzoxazol-2-ylamino)-phenylsulfanyl]-pyridin-2-carbonsäuremethylamid | 5,23E+02 | 5,15 |
| 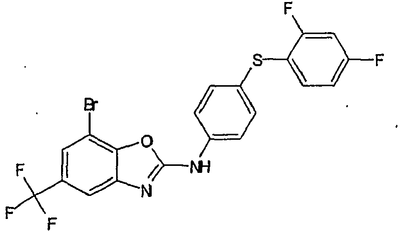 | | |
| (7-Brom-5-trifluormethyl-benzoxazol-2-yl)-[4-(2,4-difluor-phenylsulfanyl)-phenyl]-amin | 5,01 E+02 | 5,6 |

### Beispiel 3: Pharmakologische Testergebnisse

| Verbindung | Inhibierung von TIE-2 IC₅₀ (nMol) | Inhibierung von RAF IC₅₀ (nMol) |
|---|---|---|
| (5-Chlor-7-nitro-benzoxazol-2-yl)-[4-(pyridin-4-ylsulfanyl)-phenyl]-amin | 310 | >1000 |

Die nachfolgenden Beispiele betreffen pharmazeutische Zubereitungen:

### Beispiel 4: Injektionsgläser

Eine Lösung von 100 g eines erfindungsgemäßen Wirkstoffes und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel 5: Suppositorien

Man schmilzt ein Gemisch von 20 g eines erfindungsgemäßen Wirkstoffes mit 100 g Sojalecithin und 1400 g Kakaobutter, gießt in Formen und lässt erkalten. Jedes Suppositorium enthält 20 mg Wirkstoff.

### Beispiel 6: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel 7: Salbe

Man mischt 500 mg eines erfindungsgemäßen Wirkstoffes mit 99,5 g Vaseline unter aseptischen Bedingungen.

### Beispiel 8: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpresst, derart, dass jede Tablette 10 mg Wirkstoff enthält.

### Beispiel 9: Dragees

Analog Beispiel E werden Tabletten gepresst, die anschließend in üblicher Weise mit einem Überzug aus Saccharose, Kartoffelstärke, Talk, Tragant und Farbstoff überzogen werden.

### Beispiel 10: Kapseln

2 kg Wirkstoff werden in üblicher Weise in Hartgelatinekapseln gefüllt, so dass jede Kapsel 20 mg des Wirkstoffs enthält.

### Beispiel 11: Ampullen

Eine Lösung von 1 kg eines erfindungsgemäßen Wirkstoffes in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I, 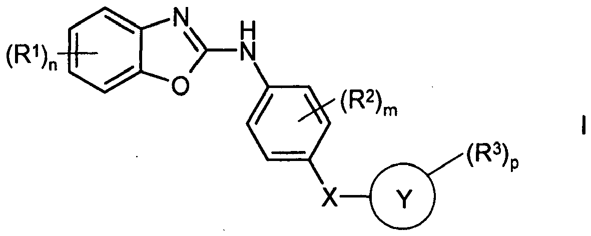 worin
R¹, R², R³ jeweils unabhängig voneinander R, Hal, CN, NO₂, NHR, NRR, NHCOR, NHSO₂R, OR, CO-R, CO-NHR, CF₃, OCF₃, SCF₃, SO₃R, SO₂R, SO₂NR, SR, COOH oder COOR,
R H,
A unverzweigtes, verzweigtes oder cyclisches Alkyl mit 1-14 C-Atomen, worin eine oder zwei CH₂-Gruppen durch O- oder S-Atome und/oder durch -CH=CH-Gruppen und/oder auch 1-7 H-Atome durch F und/oder Cl ersetzt sein können,
Ar unsubstituiertes oder ein-, zwei- oder dreifach durch A, Hal, OH, OA, CN, NO₂, NH₂, NHA, NA₂, NHCOA, SCF₃, SO₂A, COOH, COOA, CONH₂, CONHA, CONA₂, NHSO₂A, SO₂NH₂, SO₂NHA, SO₂NA₂, CHO oder COA substituiertes Phenyl, Naphthyl oder Biphenyl,
Het einen ein- oder zweikernigen gesättigten, ungesättigten oder aromatischen Heterocyclus mit 1 bis 4 N-, O-und/oder S-Atomen, der unsubstituiert oder ein-, zwei- oder dreifach durch Carbonylsauerstoff, Hal, A, -(CH₂)_{b}-Ar, -(CH₂)_{b}-Cycloalkyl, OH, OA, NH₂, NHA, NA₂, NO₂, CN, COOH, COOA, CONH₂, CONHA, CONA₂, NHCOA, NHCONH₂, NHSO₂A, CHO, COA, SO₂NH₂ und/oder S(O)_{g}A substituiert sein kann,
Hal F, Cl, Br oder I,
X O, S, SO₂NH oder NH,
Ⓨ Phenyl oder einen monocyclischen aromatischen Heterocyclus mit 1 bis 4 N-, O- und/oder S-Atomen,
b, 0, 1, 2, 3 oder 4,
g 0, 1 oder 2,
n, m, p, q jeweils unabhängig voneinander 1, 2, 3, oder 4
bedeuten,
sowie ihre pharmazeutisch unbedenklichen Salze, Solvate und Stereoisomere, einschließlich deren Mischungen in allen Verhältnissen.

2. Verbindungen nach Anspruch 1, worin
R¹ Hal, NO₂, CF₃, COOH, COOR oder H
bedeutet, sowie ihre pharmazeutisch unbedenklichen Salze, Solvate und Stereoisomere, einschließlich deren Mischungen in allen Verhältnissen.

3. Verbindungen nach Anspruch 1 oder 2, worin
R² H
bedeutet, sowie ihre pharmazeutisch unbedenklichen Salze, Solvate und Stereoisomere, einschließlich deren Mischungen in allen Verhältnissen.

4. Verbindungen nach einem oder mehreren der Ansprüche 1-3, worin
R³ H, Hal oder CO-NHR
bedeutet, sowie ihre pharmazeutisch unbedenklichen Salze, Solvate und Stereoisomere, einschließlich deren Mischungen in allen Verhältnissen.

5. Verbindungen nach einem oder mehreren der Ansprüche 1-4, worin
Y Phenyl, Furyl, Thienyl, Pyrrolyl, Imidazolyl, Pyridyl oder Pyrimidinyl
bedeutet, sowie ihre pharmazeutisch unbedenklichen Salze, Solvate und Stereoisomere, einschließlich deren Mischungen in allen Verhältnissen.

6. Verbindungen nach Anspruch 1, worin
R¹ Hal, NO₂, CF₃, COOH, COOR oder H,
R² H,
R³ H, Hal, CO-NHR,
Y Phenyl, Furyl, Thienyl, Pyrrolyl, Imidazolyl, Pyridyl oder Pyrimidinyl,
X O, S, SO₂NH oder NH,
n, p unabhängig voneinander 1, 2, 3 oder 4,
m 1,
bedeuten, sowie ihre pharmazeutisch unbedenklichen Salze, Solvate und Stereoisomere, einschließlich deren Mischungen in allen Verhältnissen.

7. Verbindungen nach Anspruch 1 ausgewählt aus der Gruppe
a) Benzoxazol-2-yl-[4-(pyridin-4-yloxy)-phenyl]-amin,
b) Benzoxazol-2-yl-[4-(pyridin-4-ylsulfanyl)-phenyl]-amin,
c) N-Benzoxazol-2-yl-N'-pyridin-4-yl-benzol-1,4-diamin,
d) 2-[4-(Pyridin-4-ylsulfanyl)-phenylamino]-benzoxazol-5-carbonsäure,
e) 2-[4-(Pyridin-4-yloxy)-phenylamino]-benzoxazol-6-carbonsäure,
f) 2-[4-(Pyridin-4-ylsulfanyl)-phenylamino]-benzoxazol-6-carbonsäure,
g) 2-[4-(Pyridin-4-ylamino)-phenylamino]-benzoxazol-6-carbonsäuremethylester,
h) (5-Nitro-benzoxazol-2-yl)-[4-(pyridin-4-ylsulfanyl)-phenyl]-amin,
i) (5-Nitro-benzoxazol-2-yl)-[4-(pyridin-4-yloxy)-phenyl]-amin,
j) N-(5-Nitro-benzoxazol-2-yl)-N'-pyridin-4-yl-benzol-1,4-diamin,
k) (6-Nitro-benzoxazol-2-yl)-[4-(pyridin-4-yloxy)-phenyl]-amin,
l) (6-Nitro-benzoxazol-2-yl)-[4-(pyridin-4-ylsulfanyl)-phenyl]-amin,
m)N-(6-Nitro-benzoxazol-2-yl)-N'-pyridin-4-yl-benzol-1,4-diamin,
n) (5-Chlor-7-nitro-benzoxazol-2-yl)-[4-(pyridin-4-yloxy)-phenyl]-amin,
o) (5-Chlor-7-nitro-benzoxazol-2-yl)-[4-(pyridin-4-ylsulfanyl)-phenyl]-amin,
p) N-(5-Chlor-7-nitro-benzoxazol-2-yl)-N'-pyridin-4-yl-benzol-1,4-diamin,
q) (7-Brom-5-trifluormethyl-benzoxazol-2-yl)-[4-(pyridin-4-yloxy)-phenyl]-amin,
r) (7-Brom-5-trifluormethyl-benzoxazol-2-yl)-[4-(pyridin-4-ylsulfanyl)-phenyl]-amin,
s) (7-Brom-5-trifluormethyl-benzoxazol-2-yl)-[4-(4-fluor-phenylsulfanyl)-phenyl]-amin,
t) N-[4-(Brom-trifluormethyl-benzoxazol-2-ylamino)-phenyl]-4-fluor-benzolsulfonamid,
u) [4-(2-Amino-6-methyl-pyrimidin-4-yloxy)-phenyl]-(7-brom-5-trifluormethyl-benzoxazol-2-yl)-amin,
v) 4-[4-(Brom-trifluormethyl-benzoxazol-2-ylamino)-phenoxy]-pyridin-2-carbonsäuremethylamid,
w) 4-[4-(Brom-trifluormethyl-benzoxazol-2-ylamino)-phenylsulfanyl]-pyridin-2-carbonsäuremethylamid,
x) (7-Brom-5-trifluormethyl-benzoxazol-2-yl)-[4-(2,4-difluor-phenylsulfanyl)-phenyl]-amin,
sowie ihre pharmazeutisch unbedenklichen Salze, Solvate und Stereoisomere, einschließlich deren Mischungen in allen Verhältnissen.

8. Verfahren zur Herstellung von Verbindungen gemäß Anspruch 1 sowie ihrer physiologisch unbedenklichen Salze, Solvate und Stereoisomere, **dadurch gekennzeichnet, dass** man eine Verbindung der Formel II, 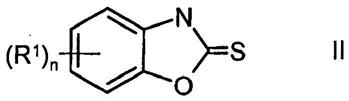 worin R¹ und n die in Anspruch 1 angegebenen Bedeutungen haben, mit einer Verbindung der Formel III, 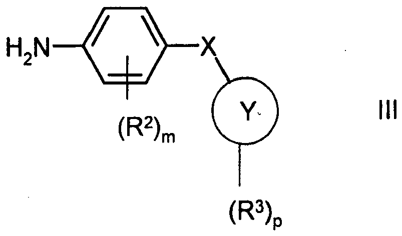 worin R², R³, X, Y, m und p die in Anspruch 1 angegebenen Bedeutungen haben, umsetzt
und/oder eine Base oder Säure der Formel I in eines ihrer Salze umwandelt.

9. Arzneimittel, enthaltend wenigstens eine Verbindung nach einem oder mehreren der Ansprüche 1-7 und/oder ihre physiologisch unbedenklichen Salze, Solvate und Stereoisomere, einschließlich deren Mischungen in allen Verhältnissen, sowie gegebenenfalls Träger- und/oder Hilfsstoffe.

10. Arzneimittel, enthaltend wenigstens eine Verbindung nach einem oder mehreren der Ansprüche 1-7 und/oder ihre physiologisch unbedenklichen Salze, Solvate und Stereoisomere, einschließlich deren Mischungen in allen Verhältnissen sowie wenigstens einen weiteren Arzneimittelwirkstoff.

11. Set (Kit) bestehend aus getrennten Packungen von
a) einer wirksamen Menge einer Verbindung nach einem oder mehreren der Ansprüche 1-7 und/oder ihrer physiologisch unbedenklichen Salze, Solvate und Stereoisomere, einschließlich deren Mischungen in allen Verhältnissen und
b) einer wirksamen Menge eines weiteren Arzneimittelwirkstoffs.

12. Verbindungen nach einem oder mehreren der Ansprüche 1-7 sowie ihre physiologisch unbedenklichen Salze, Solvate und Stereoisomere, einschließlich deren Mischungen in allen Verhältnissen als Aktivatoren oder Inhibitoren von Kinasen.

13. Verbindungen nach einem oder mehreren der Ansprüche 1-7 sowie ihre physiologisch unbedenklichen Salze, Solvate und Stereoisomere, einschließlich deren Mischungen in allen Verhältnissen als Inhibitoren von Tyrosinkinasen und/oder von Raf-Kinasen.

14. Verwendung von Verbindungen nach einem oder mehreren der Ansprüche 1-7 und/oder ihrer physiologisch unbedenklichen Salze, Solvate und Stereoisomere, einschließlich deren Mischungen in allen Verhältnissen zur Herstellung eines Medikaments zur Behandlung und/oder Prophylaxe von Krankheiten.

15. Verwendung von Verbindungen nach einem oder mehreren der Ansprüche 1-7 und/oder ihrer physiologisch unbedenklichen Salze, Solvate und Stereoisomere, einschließlich deren Mischungen in allen Verhältnissen zur Herstellung eines Medikaments zur Behandlung und/oder Prophylaxe von Krankheiten, die durch Kinasen und/oder durch kinase-vermittelte Signaltransduktion verursacht, vermittelt und/oder propagiert werden.

16. Verwendung nach Anspruch 15, wobei die Kinasen aus der Gruppe der Tyrosinkinasen ausgewählt sind.

17. Verwendung nach Anspruch 16, wobei es sich bei den Tyrosinkinasen um TIE-2 oder VEGFR handelt.

18. Verwendung nach Anspruch 15, wobei die Kinasen aus der Gruppe der Raf-Kinasen ausgewählt sind.

19. Verwendung nach Anspruch 18, wobei es sich bei den Raf-Kinasen um A-Raf, B-Raf oder Raf-1 handelt.

20. Verwendung von Verbindungen nach einem oder mehreren der Ansprüche 1-7 und/oder ihrer physiologisch unbedenklichen Salze, Solvate und Stereoisomere, einschließlich deren Mischungen in allen Verhältnissen zur Herstellung eines Medikaments zur Behandlung und/oder Prophylaxe von festen Tumoren.

21. Verwendung nach Anspruch 20, wobei der feste Tumor aus der Gruppe bestehend aus Gehirntumor, Tumor des Urogenitaltrakts, Tumor des lymphatischen Systems, Magentumor, Kehlkopftumor und Lungentumor ausgewählt ist.

22. Verwendung nach Anspruch 20, wobei der feste Tumor aus der Gruppe bestehend aus Monozytenleukämie, Lungenadenokarzinom, kleinzellige Lungenkarzinome, Bauchspeicheldrüsenkrebs, Glioblastome und Brustkarzinom ausgewählt ist.

23. Verwendung von Verbindungen nach einem oder mehreren der Ansprüche 1-7 und/oder ihrer physiologisch unbedenklichen Salze, Solvate und Stereoisomere, einschließlich deren Mischungen in allen Verhältnissen zur Herstellung eines Medikaments zur Behandlung und/oder Prophylaxe von Krankheiten, die durch Angiogenese verursacht, vermittelt und/oder propagiert werden.

24. Verwendung von Verbindungen nach einem oder mehreren der Ansprüche 1-7 und/oder ihrer physiologisch unbedenklichen Salze, Solvate und Stereoisomere, einschließlich deren Mischungen in allen Verhältnissen zur Herstellung eines Medikaments zur Behandlung und/oder Prophylaxe von Krankheiten, ausgewählt aus der Gruppe bestehend aus Retina-Vaskularisierung, diabetischer Retinopathie, altersbedingter Makula-Degeneration und/oder Entzündungskrankheiten.

25. Verwendung von Verbindungen nach einem oder mehreren der Ansprüche 1-7 und/oder ihrer physiologisch unbedenklichen Salze, Solvate und Stereoisomere, einschließlich deren Mischungen in allen Verhältnissen zur Herstellung eines Medikaments zur Behandlung und/oder Prophylaxe von Knochen-Pathologien, ausgewählt aus der Gruppe bestehend aus Osteosarkom, Osteoarthritis und Rachitis.

26. Verwendung von Verbindungen nach einem oder mehreren der Ansprüche 1-7 und/oder ihrer physiologisch unbedenklichen Salze, Solvate und Stereoisomere, einschließlich deren Mischungen in allen Verhältnissen zur Herstellung eines Medikaments zur Behandlung und/oder Prophylaxe von Krankheiten, ausgewählt aus der Gruppe bestehend aus Psoriasis, rheumatoide Arthritis, Kontaktdermatitis, Spät-Typ der Überempfindtichkeitsreaktion, Entzündungen, Endometriose, Vernarbung, gutartiger Prostatahyperplasie, immunologischer Krankheiten, Autoimmunkrankheiten und Immunschwächekrankheiten.

27. Verwendung von Verbindungen nach einem oder mehreren der Ansprüche 1-7 und/oder ihrer physiologisch unbedenklichen Salze, Solvate und Stereoisomere, einschließlich deren Mischungen in allen Verhältnissen zur Herstellung eines Medikaments zur Behandlung und/oder Prophylaxe von Krankheiten, ausgewählt aus der Gruppe bestehend aus Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischem Krebs, Schilddrüsenkrebs, Lymphom, chronischer Leukämie und akuter Leukämie.

28. Verwendung von Verbindungen nach einem oder mehreren der Ansprüche 1-7 und/oder ihrer physiologisch unbedenklichen Salze und Solvate zur Herstellung eines Arzneimittels zur Behandlung und/oder Prophylaxe von Krankheiten, wobei eine therapeutisch wirksame Menge einer Verbindung nach einem oder mehreren der Ansprüche 1-7 in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferaseinhibitoren, 7) HMG-CoA-Reduktase-Inhibitoren, 8) HIV-Protease-Inhibitoren, 9) Reverse-Transkriptase-Inhibitoren, 10) Wachstumsfaktorrezeptor-Inhibitoren sowie 11) Angiogenese-Inhibitoren verabreicht wird.

29. Verwendung von Verbindungen nach einem oder mehreren der Ansprüche 1-7 und/oder ihrer physiologisch unbedenklichen Salze und Solvate zur Herstellung eines Arzneimittels zur Behandlung und/oder Prophylaxe von Krankheiten, wobei eine therapeutisch wirksame Menge einer Verbindung nach einem oder mehreren der Ansprüche 1-7 in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferaseinhibitoren, 7) HMG-CoA-Reduktase-Inhibitoren, 8) HIV-Protease-Inhibitoren, 9) Reverse-Transkriptase-Inhibitoren, 10) Wachstumsfaktorrezeptor-Inhibitoren sowie 11) Angiogenese-Inhibitoren verabreicht wird.

## Claims

1. Compounds of the formula I, 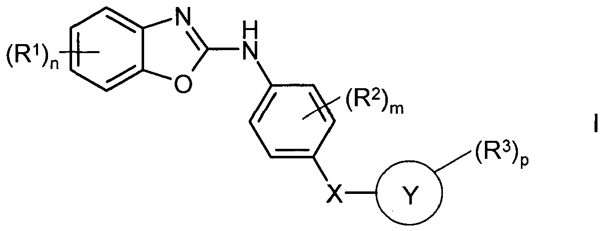 in which
R¹, R², R³ each, independently of one another, denote R, Hal, CN, NO₂, NHR, NRR, NHCOR, NHSO₂R, OR, CO-R, CO-NHR, CF₃, OCF₃, SCF₃, SO₃R, SO₂R, SO₂NR, SR, COOH or COOR,
R denotes H,
A denotes unbranched, branched or cyclic alkyl having 1-14 C atoms, in which one or two CH₂ groups may be replaced by O or S atoms and/or by -CH=CH- groups and/or in addition 1-7 H atoms may be replaced by F and/or Cl,
Ar denotes phenyl, naphthyl or biphenyl, each of which is unsubstituted or mono-, di- or trisubstituted by A, Hal, OH, OA, CN, NO₂, NH₂, NHA, NA₂, NHCOA, SCF₃, SO₂A, COOH, COOA, CONH₂, CONHA, CONA₂, NHSO₂A, SO₂NH₂, SO₂NHA, SO₂NA₂, CHO or COA,
Het denotes a mono- or bicyclic saturated, unsaturated or aromatic heterocycle having 1 to 4 N, O and/or S atoms, which may be unsubstituted or mono-, di- or tri-substituted by carbonyl oxygen, Hal, A, -(CH₂)_{b}-Ar, -(CH₂)_{b}-cycloalkyl, OH, OA, NH₂, NHA, NA₂, NO₂, CN, COOH, COOA, CONH₂, CONHA, CONA₂, NHCOA, NHCONH₂, NHSO₂A, CHO, COA, SO₂NH₂ and/or S(O)gA,
Hal denotes F, Cl, Br or I,
X denotes O, S, SO₂NH or NH,
Ⓨ denotes phenyl or a monocyclic aromatic heterocycle having 1 to 4 N, O and/or S atoms,
b denotes 0, 1, 2, 3 or 4,
g denotes 0, 1 or 2,
n, m, p, q each, independently of one another, denote 1, 2, 3 or 4,
and pharmaceutically acceptable salts, solvates and stereoisomers thereof, including mixtures thereof in all ratios.

2. Compounds according to Claim 1 in which
R¹ denotes Hal, NO₂, CF₃, COOH, COOR or H,
and pharmaceutically acceptable salts, solvates and stereoisomers thereof, including mixtures thereof in all ratios.

3. Compounds according to Claim 1 or 2 in which
R² denotes H,
and pharmaceutically acceptable salts, solvates and stereoisomers thereof, including mixtures thereof in all ratios.

4. Compounds according to one or more of Claims 1-3 in which
R³ denotes H, Hal or CO-NHR,
and pharmaceutically acceptable salts, solvates and stereoisomers thereof, including mixtures thereof in all ratios.

5. Compounds according to one or more of Claims 1-4 in which
Y denotes phenyl, furyl, thienyl, pyrrolyl, imidazolyl, pyridyl or pyrimidinyl,
and pharmaceutically acceptable salts, solvates and stereoisomers thereof, including mixtures thereof in all ratios.

6. Compounds according to Claim 1 in which
R¹ denotes Hal, NO₂, CF₃, COOH, COOR or H,
R² denotes H,
R³ denotes H, Hal, CO-NHR,
Y denotes phenyl, furyl, thienyl, pyrrolyl, imidazolyl, pyridyl or pyrimidinyl,
X denotes O, S, SO₂NH or NH,
n, p, independently of one another, denote 1, 2, 3 or 4,
m denotes 1,
and pharmaceutically acceptable salts, solvates and stereoisomers thereof, including mixtures thereof in all ratios.

7. Compounds according to Claim 1 selected from the group
a) benzoxazol-2-yl-[4-(pyridin-4-yloxy)phenyl]amine,
b) benzoxazol-2-yl-[4-(pyridin-4-ylsulfanyl)phenyl]amine,
c) N-benzoxazol-2-yl-N'-pyridin-4-ylbenzene-1,4-diamine,
d) 2-[4-(pyridin-4-ylsulfanyl)phenylamino]benzoxazole-5-carboxylic acid,
e) 2-[4-(pyridin-4-yloxy)phenylamino]benzoxazole-6-carboxylic acid,
f) 2-[4-(pyridin-4-ylsulfanyl)phenylamino]benzoxazole-6-carboxylic acid,
g) methyl 2-[4-(pyridin-4-ylamino)phenylamino]benzoxazole-6-carboxylate,
h) (5-nitrobenzoxazol-2-yl)-[4-(pyridin-4-ylsulfanyl)phenyl]amine,
i) (5-nitrobenzoxazol-2-yl)-[4-(pyridin-4-yloxy)phenyl]amine,
j) N-(5-nitrobenzoxazol-2-yl)-N'-pyridin-4-ylbenzene-1,4-diamine,
k) (6-nitrobenzoxazol-2-yl)-[4-(pyridin-4-yloxy)phenyl]amine,
l) (6-nitrobenzoxazol-2-yl)-[4-(pyridin-4-ylsulfanyl)phenyl]amine,
m)N-(6-nitrobenzoxazol-2-yl)-N'-pyridin-4-ylbenzene-1,4-diamine,
n) (5-chloro-7-nitrobenzoxazol-2-yl)-[4-(pyridin-4-yloxy)phenyl]amine,
o) (5-chloro-7-nitrobenzoxazol-2-yl)-[4-(pyridin-4-ylsulfanyl)phenyl]-amine,
p) N-(5-chloro-7-nitrobenzoxazol-2-yl)-N'-pyridin-4-ylbenzene-1,4-di-amine,
q) (7-bromo-5-trifluoromethylbenzoxazol-2-yl)-[4-(pyridin-4-yloxy)-phenyl]amine,
r) (7-bromo-5-trifluoromethylbenzoxazol-2-yl)-[4-(pyridin-4-ylsulfanyl)-phenyl]amine,
s) (7-bromo-5-trifluoromethylbenzoxazol-2-yl)-[4-(4-fluorophenylsulfanyl)phenyl]amine,
t) N-[4-(bromotrifluoromethylbenzoxazol-2-ylamino)phenyl]-4-fluoro-benzenesulfonamide,
u) [4-(2-amino-6-methylpyrimidin-4-yloxy)phenyl]-(7-bromo-5-trifluoro-methylbenzoxazol-2-yl)amine,
v) N-methyl-4-[4-(bromotrifluoromethylbenzoxazol-2-ylamino)-phenoxy]pyridine-2-carboxamide,
w) N-methyl-4-[4-(bromotrifluoromethylbenzoxazol-2-ylamino)phenyl-sulfanyl]pyridine-2-carboxamide,
x) (7-bromo-5-trifluoromethylbenzoxazol-2-yl)-[4-(2,4-difluorophenyl-sulfanyl)phenyl]amine,
and pharmaceutically acceptable salts, solvates and stereoisomers thereof, including mixtures thereof in all ratios.

8. Process for the preparation of compounds according to Claim 1and physiologically acceptable salts, solvates and stereoisomers thereof, **characterised in that**
a compound of the formula II, 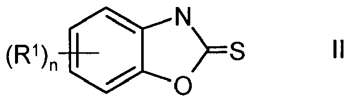 in which R¹ and n have the meanings indicated in Claim 1,
is reacted with a compound of the formula III, 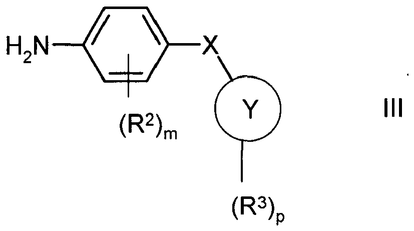 in which R², R³, X, Y, m and p have the meanings indicated in Claim 1,
and/or a base or acid of the formula I is converted into one of its salts.

9. Medicaments comprising at least one compound according to one or more of Claims 1-7 and/or physiologically acceptable salts, solvates and stereoisomers thereof, including mixtures thereof in all ratios, and optionally excipients and/or adjuvants.

10. Medicaments comprising at least one compound according to one or more of Claims 1-7 and/or physiologically acceptable salts, solvates and stereoisomers thereof, including mixtures thereof in all ratios, and at least one further medicament active compound.

11. Set (kit) consisting of separate packs of
a) an effective amount of a compound according to one or more of Claims 1-7 and/or physiologically acceptable salts, solvates and stereoisomers thereof, including mixtures thereof in all ratios, and
b) an effective amount of a further medicament active compound.

12. Compounds according to one or more of Claims 1-7 and physiologically acceptable salts, solvates and stereoisomers thereof, including mixtures thereof in all ratios, as activators or inhibitors of kinases.

13. Compounds according to one or more of Claims 1-7 and physiologically acceptable salts, solvates and stereoisomers thereof, including mixtures thereof in all ratios, as inhibitors of tyrosine kinases and/or of Raf kinases.

14. Use of compounds according to one or more of Claims 1-7 and/or physiologically acceptable salts, solvates and stereoisomers thereof, including mixtures thereof in all ratios, for the preparation of a medicament for the treatment and/or prophylaxis of diseases.

15. Use of compounds according to one or more of Claims 1-7 and/or physiologically acceptable salts, solvates and stereoisomers thereof, including mixtures thereof in all ratios, for the preparation of a medicament for the treatment and/or prophylaxis of diseases that are caused, mediated and/or propagated by kinases and/or by kinase-mediated signal transduction.

16. Use according to Claim 15, where the kinases are selected from the group of the tyrosine kinases.

17. Use according to Claim 16, where the tyrosine kinases are TIE-2 or VEGFR.

18. Use according to Claim 15, where the kinases are selected from the group of the Raf kinases.

19. Use according to Claim 18, where the Raf kinases are A-Raf, B-Raf or Raf-1.

20. Use of compounds according to one or more of Claims 1-7 and/or physiologically acceptable salts, solvates and stereoisomers thereof, including mixtures thereof in all ratios, for the preparation of a medicament for the treatment and/or prophylaxis of solid tumours.

21. Use according to Claim 20, where the solid tumour is selected from the group consisting of brain tumour, tumour of the urogenital tract, tumour of the lymphatic system, stomach tumour, laryngeal tumour and lung tumour.

22. Use according to Claim 20, where the solid tumour is selected from the group consisting of monocytic leukaemia, lung adenocarcinoma, small cell lung carcinomas, pancreatic cancer, glioblastomas and breast carcinoma.

23. Use of compounds according to one or more of Claims 1-7 and/or physiologically acceptable salts, solvates and stereoisomers thereof, including mixtures thereof in all ratios, for the preparation of a medicament for the treatment and/or prophylaxis of diseases that are caused, mediated and/or propagated by angiogenesis.

24. Use of compounds according to one or more of Claims 1-7 and/or physiologically acceptable salts, solvates and stereoisomers thereof, including mixtures thereof in all ratios, for the preparation of a medicament for the treatment and/or prophylaxis of diseases selected from the group consisting of retinal vascularisation, diabetic retino-pathy, age-induced macular degeneration and/or inflammatory diseases.

25. Use of compounds according to one or more of Claims 1-7 and/or physiologically acceptable salts, solvates and stereoisomers thereof, including mixtures thereof in all ratios, for the preparation of a medicament for the treatment and/or prophylaxis of bone pathologies selected from the group consisting of osteosarcoma, osteoarthritis and rickets.

26. Use of compounds according to one or more of Claims 1-7 and/or physiologically acceptable salts, solvates and stereoisomers thereof, including mixtures thereof in all ratios, for the preparation of a medicament for the treatment and/or prophylaxis of diseases selected from the group consisting of psoriasis, rheumatoid arthritis, contact dermatitis, delayed hypersensitivity reaction, inflammation, endometriosis, scarring, benign prostatic hyperplasia, immunological diseases, autoimmune diseases and immunodeficiency diseases.

27. Use of compounds according to one or more of Claims 1-7 and/or physiologically acceptable salts, solvates and stereoisomers thereof, including mixtures thereof in all ratios, for the preparation of a medicament for the treatment and/or prophylaxis of diseases selected from the group consisting of brain cancer, lung cancer, squamous cell cancer, bladder cancer, stomach cancer, pancreatic cancer, liver cancer, kidney cancer, colorectal cancer, breast cancer, head cancer, neck cancer, oesophageal cancer, gynaecological cancer, thyroid cancer, lymphoma, chronic leukaemia and acute leukaemia.

28. Use of compounds according to one or more of Claims 1-7 and/or physiologically acceptable salts and solvates thereof for the preparation of a medicament for the treatment and/or prophylaxis of diseases, where a therapeutically effective amount of a compound according to one or more of Claims 1-7 is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitors, 7) HMG-CoA reductase inhibitors, 8) HIV protease inhibitors, 9) reverse transcriptase inhibitors, 10) growth factor receptor inhibitors and 11) angiogenesis inhibitors.

29. Use of compounds according to one or more of Claims 1-7 and/or physiologically acceptable salts and solvates thereof for the preparation of a medicament for the treatment and/or prophylaxis of diseases, where a therapeutically effective amount of a compound according to one or more of Claims 1-7 is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitors, 7) HMG-CoA reductase inhibitors, 8) HIV protease inhibitors, 9) reverse transcriptase inhibitors, 10) growth factor receptor inhibitors and 11) angiogenesis inhibitors.

## Revendications

1. Composés de formule I, 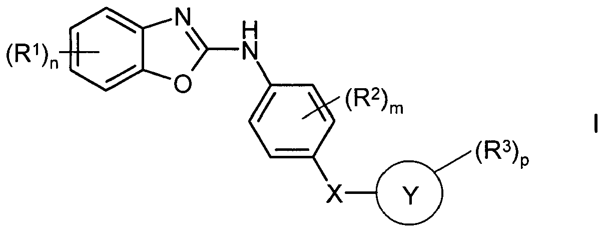 dans laquelle
R¹, R², R³ désignent chacun, indépendamment les uns des autres, R, Hal, CN, NO₂, NHR, NRR, NHCOR, NHSO₂R, OR, CO-R, CO-NHR, CF₃, OCF₃, SCF₃, SO₃R, SO₂R, SO₂NR, SR, COOH ou COOR,
R désigne H,
A désigne alkyle non ramifié, ramifié ou cyclique ayant 1-14 atomes de C, où un ou deux groupements CH₂ peuvent être remplacés par des atomes de O ou S et/ou par des groupements -CH=CH- et/ou de plus 1-7 atomes de H peuvent être remplacés par F et/ou CI,
Ar désigne phényle, naphtyle ou biphényle, chacun d'entre eux étant non substitué ou mono-, di- ou trisubstitué par A, Hal, OH, OA, CN, NO₂, NH₂, NHA, NA₂, NHCOA, SCF₃, SO₂A, COOH, COOA, CONH₂, CONHA, CONA₂, NHSO₂A, SO₂NH₂, SO₂NHA, SO₂NA₂, CHO ou COA,
Hét désigne un hétérocycle mono- ou bicyclique saturé, insaturé ou aromatique ayant de 1 à 4 atomes de N, O et/ou S, pouvant être non substitué ou mono-, di- ou tri-substitué par oxygène de carbonyle, Hal, A, -(CH₂)_{b}-Ar, -(CH₂)_{b}-cycloalkyle, OH, OA, NH₂, NHA, NA₂, NO₂, CN, COOH, COOA, CONH₂, CONHA, CONA₂, NHCOA, NHCONH₂, NHSO₂A, CHO, COA, SO₂NH₂ et/ou S(O)_{g}A,
Hal désigne F, Cl, Br ou I,
X désigne O, S, SO₂NH ou NH,
Ⓨ désigne phényle ou un hétérocycle monocyclique aromatique ayant de 1 à 4 atomes de N, O et/ou S,
b désigne 0, 1, 2, 3 ou 4,
g désigne 0, 1 ou 2,
n, m, p, q désignent chacun, indépendamment les uns des autres, 1, 2, 3 ou 4,
et les sels, solvats et stéréoisomères pharmaceutiquement acceptables de ceux-ci, y compris des mélanges de ceux-ci en toutes proportions.

2. Composés selon la revendication 1, dans lesquels
R¹ désigne Hal, NO₂, CF₃, COOH, COOR ou H,
et les sels, solvats et stéréoisomères pharmaceutiquement acceptables de ceux-ci, y compris des mélanges de ceux-ci en toutes proportions.

3. Composés selon la revendication 1 ou 2, dans lesquels
R² désigne H,
et les sels, solvats et stéréoisomères pharmaceutiquement acceptables de ceux-ci, y compris des mélanges de ceux-ci en toutes proportions.

4. Composés selon l'une ou plusieurs parmi les revendications 1-3, dans lesquels
R³ désigne H, Hal ou CO-NHR,
et les sels, solvats et stéréoisomères pharmaceutiquement acceptables de ceux-ci, y compris des mélanges de ceux-ci en toutes proportions.

5. Composés selon l'une ou plusieurs parmi les revendications 1-4, dans lesquels
Y désigne phényle, furyle, thiényle, pyrrolyle, imidazolyle, pyridyle ou pyrimidinyle,
et les sels, solvats et stéréoisomères pharmaceutiquement acceptables de ceux-ci, y compris des mélanges de ceux-ci en toutes proportions.

6. Composés selon la revendication 1, dans lesquels
R¹ désigne Hal, NO₂, CF₃, COOH, COOR ou H,
R² désigne H,
R³ désigne H, Hal, CO-NHR,
Y désigne phényle, furyle, thiényle, pyrrolyle, imidazolyle, pyridyle ou pyrimidinyle,
X désigne O, S, SO₂NH ou NH,
n, p, désignent, indépendamment l'un de l'autre, 1, 2, 3 ou 4,
m désigne 1,
et les sels, solvats et stéréoisomères pharmaceutiquement acceptables de ceux-ci, y compris des mélanges de ceux-ci en toutes proportions.

7. Composés selon la revendication 1, choisis dans le groupe constitué par
a) la benzoxazol-2-yl-[4-(pyridin-4-yloxy)phényl]amine,
b) la benzoxazol-2-yl-[4-(pyridin-4-ylsulfanyl)phényl]amine,
c) la N-benzoxazol-2-yl-N'-pyridin-4-ylbenzène-1,4-diamine,
d) l'acide 2-[4-(pyridin-4-ylsulfanyl)phénylamino]benzoxazole-5-carboxylique,
e) l'acide 2-[4-(pyridin-4-yloxy)phénylamino]benzoxazole-6-carboxylique,
f) l'acide 2-[4-(pyridin-4-ylsulfanyl)phénylamino]benzoxazole-6-carboxylique,
g) le 2-[4-(pyridin-4-ylamino)phénylamino]benzoxazole-6-carboxylate de méthyle,
h) la (5-nitrobenzoxazol-2-yl)-[4-(pyridin-4-ylsulfanyl)phényl]amine,
i) la (5-nitrobenzoxazol-2-yl)-[4-(pyridin-4-yloxy)phényl]amine,
j) la N-(5-nitrobenzoxazol-2-yl)-N'-pyridin-4-ylbenzène-1,4-diamine,
k) la (6-nitrobenzoxazol-2-yl)-[4-(pyridin-4-yloxy)phényl]amine,
l) la (6-nitrobenzoxazol-2-yl)-[4-(pyridin-4-ylsulfanyl)phényl]amine,
m) la N-(6-nitrobenzoxazol-2-yl)-N'-pyridin-4-ylbenzène-1,4-diamine,
n) la (5-chloro-7-nitrobenzoxazol-2-yl)-[4-(pyridin-4-yloxy)phényl]-amine,
o) la (5-chloro-7-nitrobenzoxazol-2-yl)-[4-(pyridin-4-ylsulfanyl)phényl]-amine,
p) la N-(5-chloro-7-nitrobenzoxazol-2-yl)-N'-pyridin-4-ylbenzène-1,4-diamine,
q) la (7-bromo-5-trifluorométhylbenzoxazol-2-yl)-[4-(pyridin-4-yloxy)-phényl]amine,
r) la (7-bromo-5-trifluorométhylbenzoxazol-2-yl)-[4-(pyridin-4-yl-sulfanyl)phényl]amine,
s) la (7-bromo-5-trifluorométhylbenzoxazol-2-yl)-[4-(4-fluorophényl-sulfanyl)phényl]amine,
t) le N-[4-(bromotrifluorométhylbenzoxazol-2-ylamino)phényl]-4-fluorobenzènesulfonamide,
u) la [4-(2-amino-6-méthylpyrimidin-4-yloxy)phényl]-(7-bromo-5-tri-fluorométhylbenzoxazol-2-yl)amine,
v) le N-méthyl-4-[4-(bromotrifluorométhylbenzoxazol-2-ylamino)-phénoxy]pyridine-2-carboxamide,
w) le N-méthyl-4-[4-(bromotrifluorométhylbenzoxazol-2-ylamino)-phénylsulfanyl]pyridine-2-carboxamide,
x) la (7-bromo-5-trifluorométhylbenzoxazol-2-yl)-[4-(2,4-difluoro-phénylsulfanyl)phényl]amine,
et les sels, solvats et stéréoisomères pharmaceutiquement acceptables de ceux-ci, y compris des mélanges de ceux-ci en toutes proportions.

8. Procédé de préparation de composés selon la revendication 1 et de sels, solvats et stéréoisomères physiologiquement acceptables de ceux-ci, **caractérisé en ce que**
un composé de formule II, 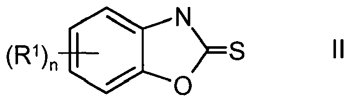 dans laquelle R¹ et n revêtent les significations indiquées selon la revendication 1,
est réagi avec un composé de formule III, 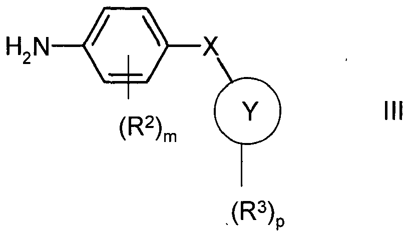 dans laquelle R², R³, X, Y, m et p revêtent les significations indiquées selon la revendication 1,
et/ou une base ou un acide de formule I est converti(e) en l'un de ses sels.

9. Médicaments comprenant au moins un composé selon l'une ou plusieurs parmi les revendications 1-7 et/ou des sels, solvats et stéréoisomères physiologiquement acceptables de celui-ci, y compris des mélanges de ceux-ci en toutes proportions, et éventuellement des excipients et/ou des adjuvants.

10. Médicaments comprenant au moins un composé selon l'une ou plusieurs parmi les revendications 1-7 et/ou des sels, solvats et stéréoisomères physiologiquement acceptables de ceux-ci, y compris des mélanges de ceux-ci en toutes proportions, et au moins un autre composé actif médicamenteux.

11. Ensemble (kit) constitué de conditionnements séparés
a) d'une quantité efficace d'un composé selon l'une ou plusieurs parmi les revendications 1-7 et/ou de sels, solvats et stéréoisomères physiologiquement acceptables de celui-ci, y compris des mélanges de ceux-ci en toutes proportions, et
b) d'une quantité efficace d'un autre composé actif médicamenteux.

12. Composés selon l'une ou plusieurs parmi les revendications 1-7 et des sels, solvats et stéréoisomères physiologiquement acceptables de ceux-ci, y compris des mélanges de ceux-ci en toutes proportions, comme activateurs ou inhibiteurs de kinases.

13. Composés selon l'une ou plusieurs parmi les revendications 1-7 et des sels, solvats et stéréoisomères physiologiquement acceptables de ceux-ci, y compris des mélanges de ceux-ci en toutes proportions, comme inhibiteurs de tyrosine kinases et/ou de Raf kinases.

14. Utilisation de composés selon l'une ou plusieurs parmi les revendications 1-7 et/ou de sels, solvats et stéréoisomères physiologiquement acceptables de ceux-ci, y compris des mélanges de ceux-ci en toutes proportions, pour la préparation d'un médicament destiné au traitement et/ou à la prophylaxie de maladies.

15. Utilisation de composés selon l'une ou plusieurs parmi les revendications 1-7 et/ou de sels, solvats et stéréoisomères physiologiquement acceptables de ceux-ci, y compris des mélanges de ceux-ci en toutes proportions, pour la préparation d'un médicament destiné au traitement et/ou à la prophylaxie de maladies qui sont provoquées, médiées et/ou propagées par des kinases et/ou par une transduction de signaux médiée par des kinases.

16. Utilisation selon la revendication 15, dans laquelle les kinases sont choisies dans le groupe constitué par les tyrosine kinases.

17. Utilisation selon la revendication 16, dans laquelle les tyrosine kinases sont TIE-2 ou VEGFR.

18. Utilisation selon la revendication 15, dans laquelle les kinases sont choisies dans le groupe constitué par les Raf kinases.

19. Utilisation selon la revendication 18, dans laquelle les Raf kinases sont A-Raf, B-Raf ou Raf-1.

20. Utilisation de composés selon l'une ou plusieurs parmi les revendications 1-7 et/ou de sels, solvats et stéréoisomères physiologiquement acceptables de ceux-ci, y compris des mélanges de ceux-ci en toutes proportions, pour la préparation d'un médicament destiné au traitement et/ou à la prophylaxie de tumeurs solides.

21. Utilisation selon la revendication 20, dans laquelle la tumeur solide provient du groupe constitué par une tumeur du cerveau, une tumeur du tractus urogénital, une tumeur du système lymphatique, une tumeur de l'estomac, une tumeur du larynx et une tumeur du poumon.

22. Utilisation selon la revendication 20, dans laquelle la tumeur solide provient du groupe constitué par la leucémie monocytique, l'adénocarcinome du poumon, les carcinomes du poumon à petites cellules, le cancer du pancréas, les glioblastomes et le carcinome du sein.

23. Utilisation de composés selon l'une ou plusieurs parmi les revendications 1-7 et/ou de sels, solvats et stéréoisomères physiologiquement acceptables de ceux-ci, y compris des mélanges de ceux-ci en toutes proportions, pour la préparation d'un médicament destiné au traitement et/ou à la prophylaxie de maladies qui sont provoquées, médiées et/ou propagées par l'angiogenèse.

24. Utilisation de composés selon l'une ou plusieurs parmi les revendications 1-7 et/ou de sels, solvats et stéréoisomères physiologiquement acceptables de ceux-ci, y compris des mélanges de ceux-ci en toutes proportions, pour la préparation d'un médicament destiné au traitement et/ou à la prophylaxie de maladies choisies dans le groupe constitué par la vascularisation rétinienne, la rétinopathie diabétique, la dégénérescence maculaire liée à l'âge et/ou les maladies inflammatoires.

25. Utilisation de composés selon l'une ou plusieurs parmi les revendications 1-7 et/ou de sels, solvats et stéréoisomères physiologiquement acceptables de ceux-ci, y compris des mélanges de ceux-ci en toutes proportions, pour la préparation d'un médicament destiné au traitement et/ou à la prophylaxie de pathologies osseuses choisies dans le groupe constitué par l'ostéosarcome, l'arthrose et le rachitisme.

26. Utilisation de composés selon l'une ou plusieurs parmi les revendications 1-7 et/ou de sels, solvats et stéréoisomères physiologiquement acceptables de ceux-ci, y compris des mélanges de ceux-ci en toutes proportions, pour la préparation d'un médicament destiné au traitement et/ou à la prophylaxie de maladies choisies dans le groupe constitué par le psoriasis, la polyarthrite rhumatoïde, la dermite de contact, la réaction d'hypersensibilité retardée, les inflammations, l'endométriose, la cicatrisation, l'hyperplasie bénigne de la prostate, les maladies immunologiques, les maladies auto-immunes et les maladies d'immunodéficience.

27. Utilisation de composés selon l'une ou plusieurs parmi les revendications 1-7 et/ou de sels, solvats et stéréoisomères physiologiquement acceptables de ceux-ci, y compris des mélanges de ceux-ci en toutes proportions, pour la préparation d'un médicament destiné au traitement et/ou à la prophylaxie de maladies choisies dans le groupe constitué par le cancer du cerveau, le cancer du poumon, le cancer à cellules squameuses, le cancer de la vessie, le cancer de l'estomac, le cancer du pancréas, le cancer du foie, le cancer du rein, le cancer colorectal, le cancer du sein, le cancer de la tête, le cancer du cou, le cancer de l'oesophage, le cancer gynécologique, le cancer de la thyroïde, le lymphome, la leucémie chronique et la leucémie aiguë.

28. Utilisation de composés selon l'une ou plusieurs parmi les revendications 1-7 et/ou de sels et solvats physiologiquement acceptables de ceux-ci pour la préparation d'un médicament destiné au traitement et/ou à la prophylaxie de maladies, dans laquelle une quantité thérapeutiquement efficace d'un composé selon l'une ou plusieurs parmi les revendications 1-7 est administrée en association avec un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) des inhibiteurs de la prényl-protéine transférase, 7) des inhibiteurs de la HMG-CoA réductase, 8) des inhibiteurs de la protéase du VIH, 9) des inhibiteurs de la transcriptase inverse, 10) des inhibiteurs du récepteur du facteur de croissance et 11) des inhibiteurs de l'angiogenèse.

29. Utilisation de composés selon l'une ou plusieurs parmi les revendications 1-7 et/ou de sels et solvats physiologiquement acceptables de ceux-ci pour la préparation d'un médicament destiné au traitement et/ou à la prophylaxie de maladies, dans laquelle une quantité thérapeutiquement efficace d'un composé selon l'une ou plusieurs parmi les revendications 1-7 est administrée en association avec une radiothérapie et un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) des inhibiteurs de la prényl-protéine transférase, 7) des inhibiteurs de la HMG-CoA réductase, 8) des inhibiteurs de la protéase du VIH, 9) des inhibiteurs de la transcriptase inverse, 10) des inhibiteurs du récepteur du facteur de croissance et 11) des inhibiteurs de l'angiogenèse.
